# EUROPEAN PATENT APPLICATION

(11) **EP 1 746 108 A1**
(43) Date of publication of application: **24.01.2007**
(21) Application number: 05736922.5
(22) Date of filing: 25.04.2005
(51) Int. Cl.: C08B 37/08, A61K 31/737, A61P 7/02, A61P 25/00, A61P 25/28, A61K 35/60

(54) **FISH-ORIGIN CHONDROITIN SULFATE/DERMATAN SULFATE HYBRID CHAIN**

(30) Priority: 27.04.2004 US 565511 P; 22.10.2004 JP 2004308584
(71) Applicant: MARUHA CORPORATION, Chiyoda-ku, Tokyo 100-8608 (JP); Sugahara, Kazuyuki, Sakyo-ku Kyoto-shi, Kyoto 606-8415 (JP)
(72) Inventor: SUGAHARA, Kazuyuki, Kyoto-shi, Kyoto 606-8415 (JP)
(74) Representative: Ziebig, Marlene
(86) International application number: PCT/JP2005/008326
(87) International publication number: WO 2005/103089

(57) **Abstract**

It is intended to provide a novel chondroitin sulfate/dermatan sulfate hybrid chain having biding activity toward a variety of growth factors, neurite outgrowth-promoting activity, and anticoagulant activity and to provide a composition for neurological disease treatment. The novel chondroitin sulfate/dermatan sulfate hybrid chain was separated and purified from the bodies of fishes other than bony fishes. When shark skin is used, the hybrid chain comprises a disaccharide GlcUA(2S)-GalNAc(4S) and has an average molecular weight of 65 to 75 kDa, and the degree of sulfation per disaccharide molecule falls within the range of 0.7 or more to less than 1.20. The hybrid chain is capable of binding to a variety of growth factors and further possesses neurite outgrowth-promoting activity and anticoagulant activity.

## Description

### Technical Field

The present invention relates to a chondroitin sulfate/dermatan sulfate hybrid chain derived from fishes, particularly shark skin (SS-CS/DS) or hagfish notochord (HN-CS/DS).

### Background Art

Animal tissues have a group of sulfated sugar chains called glycosaminoglycans, which exist as proteoglycan molecules covalently linked to proteins and play essential roles in cell growth and differentiation, tissue morphogenesis, and so on (Lewandowska, K., Choi, H. U., Rosenberg, L. C., Zardi, L., and Culp, L. A. (1987) J. Cell Biol. 105, 1443-1454; Yamaguchi, Y., Mann, D. M., and Ruoslahti, E. (1990) Nature, 346, 281-284; Lyon, M., Deakin, J. A., Rahmoune, H., Fernig, D. G., Nakamura, T., and Gallagher, J. T. (1998) J. Biol. Chem. 273, 271-278; and Trowbridge, J. M., and Gallo, R. L. (2002) Glycobiology 12, 117R-125R).

Among glycosaminoglycans, chondroitin sulfate is composed of a disaccharide (D-glucuronic acid and N-acetyl-D-galactosamine) and a sulfate residue. Chondroitin sulfate is classified into types such as A, C, D, E, and K according to the binding position of the sulfate group. Chondroitin sulfate B contains L-iduronic acid instead of D-glucuronic acid as a constituent sugar and is called dermatan sulfate.

Chondroitin sulfate and dermatan sulfate contain many overlapping sequences composed of disaccharide units modified by different sulfation patterns, exhibiting enormous structural diversity comparable to heparan sulfate.

The present inventors have been engaged in the elucidation of participation of a variety of oversulfated chondroitin sulfate and dermatan sulfate chains in neural development. As a result, the present inventors have turned the spotlight on the importance of oversulfated disaccharide units "D" unit [GlcUA(2S)-GalNAc(6S)], "iD" unit [IdoUA(2S)-GalNAc(6S)], "E" unit [GlcUA-GalNAc(4S,6S)], and "iE" unit [IdoUA-GalNAc(4S,6S)] in chondroitin sulfate and dermatan sulfate having various biological functions, (wherein IdoUA represents L-iduronic acid, GlcUA represents D-glucuronic acid, GalNAc represents N-acetyl-D-galactosamine, and 2S, 4S, and 6S represent the binding sites of the sulfate group, that is, C-2 position, C-4 position, and C-6 position, respectively).

Each neuron develops characteristic morphology during the formation of neural circuit while extending its axon along a specific pathway, thereby finding a correct target cell to form synapse therewith. Many researchers have previously found that the chondroitinase ABC treatment of brain tissues in the developmental stage to decompose and remove chondroitin sulfate contained in the tissues causes severe abnormalities in neuronal morphogenesis and axonal course. From such observation, chondroitin sulfate has been thought to participate in the induction of axons of neurites or participate in neuronal morphogenesis.

The present inventors have cultured hippocampal neurons prepared from embryonic day 16 mice on substrates coated with a variety of chondroitin sulfate (dermatan sulfate) preparations, and have thereby found that sugar chains having a particular structure exhibit neurite outgrowth-promoting activity. Particularly, the present inventors have found that those containing D or iD unit in large amounts extend dendrite-like processes while those rich in E or iE unit extend long axon-like processes (see Hikino, M., Mikami, T., Faissner, A., Vilela-Silva, A. C., Pavao, M. S., and Sugahara, K. (2003) J. Biol. Chem. 278, 43744-43754; and Sugahara, K and Yamada, S., Trends in Glycoscinence and Glycotechnology vol. 12 No. 67 pp. 321-349).

Such oversulfated chondroitin sulfate and dermatan sulfate are known to bind to various heparin-binding growth factors, suggesting that their signal transduction is involved in the morphogenesis of hippocampal neurons.

From these facts, it has been expected that a source of glycosaminoglycan with unique structures and remarkable activity, which is useful for use as therapeutic agents for neurological diseases, is searched. As already reported, mucopolysaccharide has been purified from the skins of marine organisms, for example shark (Seno, N. and Meyer, K., Biochim. Biophys. Acta. 78 (1963) 258-264).
[Non-Patent Document 1]: Hikino, M., Mikami, T., Faissner, A., Vilela-Silva, A. C., Pavao, M. S., and Sugahara, K. (2003) J. Biol. Chem. 278, 43744-43754
[Non-Patent Document 2]: Seno, N. and Meyer, K., Biochim. Biophys. Acta. 78 (1963) 258-264
[Non-Patent Document 3]: Sugahara, K and Yamada, S., Trends in Glycoscience and Glycotechnology vol. 12 No. 67 pp. 321-349

### Disclosure of the Invention

An object of the present invention is to provide a chondroitin sulfate/dermatan sulfate hybrid chain having binding activity toward a variety of growth factors, neurite outgrowth-promoting activity, and anticoagulant activity, which is derived from fishes, for example fishes except for bony fishes, particularly shark skin or hagfish notochord, and to provide a composition for neurological disease treatment.

The present inventors have found that a fraction of a novel chondroitin sulfate/dermatan sulfate hybrid chain separated and purified from a portion of the bodies of fishes except for bony fishes such as shark skin binds to a variety of growth factors and further possesses neurite outgrowth-promoting activity and anticoagulant activity. Furthermore, the hybrid chain can be expected to be effective as a composition for neurological disease treatment. Thus, the present invention relates to the following aspects:
1. A chondroitin sulfate/dermatan sulfate hybrid chain comprising a disaccharide GlcUA(2S)-GalNAc(4S) (B unit);
2. The chondroitin sulfate/dermatan sulfate hybrid chain according to 1, wherein the chain comprises GlcUA(2S)-GalNAc(4S) (B unit) and further comprises at least one unit selected from the group consisting of GlcUA-GalNAc(4S) (A unit), GlcUA-GalNAc(6S) (C unit), GlcUA(2S)-GalNAc(6S) (D unit), GlcUA-GalNAc(4S,6S) (E unit), and unsulfated GlcUA-GalNAc (O unit) and at least one unit selected from the group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), and IdoUA-GalNAc(4S,6S) (iE unit);
3. The chondroitin sulfate/dermatan sulfate hybrid chain according to 1 or 2, wherein an average molecular weight thereof is 65 to 75 kDa;
4. The chondroitin sulfate/dermatan sulfate hybrid chain according to any one of 1 to 3, wherein the degree of sulfation per disaccharide molecule falls within the range of 0.70 or more to less than 1.2;
5. The chondroitin sulfate/dermatan sulfate hybrid chain according to any one of 1 to 4, wherein the chain is derived from shark skin;
6. Glycosaminoglycan comprising a chondroitin sulfate/dermatan sulfate hybrid chain according to any one of 1 to 5;
7. A growth factor-binding agent comprising glycosaminoglycan according to 6 as an active ingredient;
8. A neurite outgrowth-promoting agent comprising glycosaminoglycan according to 6 as an active ingredient;
9. An anticoagulant comprising glycosaminoglycan according to 6 as an active ingredient;
10. A pharmaceutical composition comprising glycosaminoglycan according to 6 as an active ingredient;
11. A pharmaceutical composition for neurodegenerative disease prevention or treatment comprising glycosaminoglycan according to 6 as an active ingredient;
12. Use of glycosaminoglycan according to 6 for producing a growth factor-binding agent, neurite outgrowth-promoting agent, anticoagulant, or composition for neurodegenerative disease treatment;
13. Glycosaminoglycan comprising a chondroitin sulfate/dermatan sulfate hybrid chain derived from fishes except for bony fishes;
14. The glycosaminoglycan comprising a chondroitin sulfate/dermatan sulfate hybrid chain according to 13, wherein the chain comprises at least one unit selected from the group consisting of GlcUA-GalNAc(4S) (A unit), GlcUA(2S)-GalNAc(4S) (B unit), GlcUA-GalNAc(6S) (C unit), GlcUA(2S)-GalNAc(6S) (D unit), GlcUA-GalNAc(4S,6S) (E unit), GlcUA(2S)β1-3GalNAc(4S,6S) (T unit), and unsulfated GlcUA-GalNAc (O unit) and at least one unit selected from the group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), IdoUA-GalNAc(4S,6S) (iE unit), and IdoUA(2S) α1-3GalNAc(4S,6S) (iT unit);
15. A growth factor-binding agent comprising glycosaminoglycan according to 13 or 14 as an active ingredient;
16. A neurite outgrowth-promoting agent comprising glycosaminoglycan according to 13 or 14 as an active ingredient;
17. A composition for neurodegenerative disease treatment comprising glycosaminoglycan according to 13 or 14 as an active ingredient;
18. A pharmaceutical composition comprising glycosaminoglycan according to 13 or 14 as an active ingredient;
19. A pharmaceutical composition for the prevention or treatment of neurodegenerative disease comprising glycosaminoglycan according to 13 or 14 as an active ingredient; and
20. Use of glycosaminoglycan according to 13 or 14 for producing a growth factor-binding agent, neurite outgrowth-promoting agent, or composition for neurodegenerative disease treatment.

The present specification encompasses contents described in the specifications and/or drawings of Japanese Patent Application No. 2004-308584 and US provisional Application No. 60/565,511 that serve as a basis of the priority of the present application.

### Brief Description of the Drawings

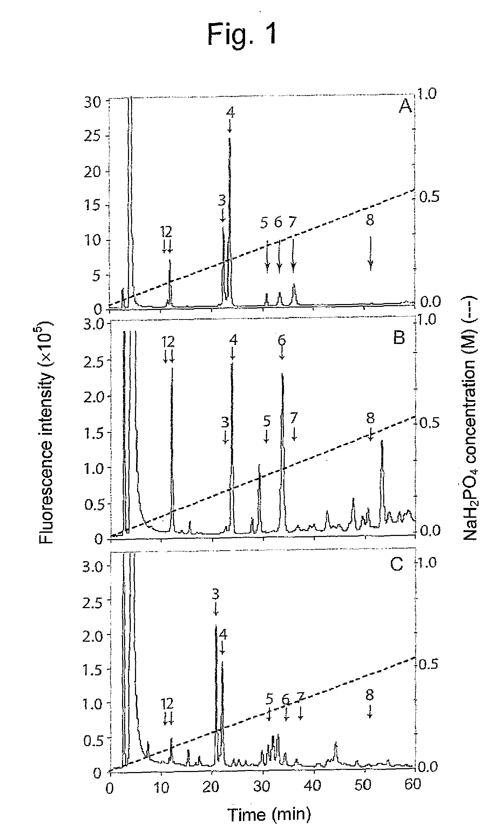

Figure 1 is a diagram showing an analysis result of chondroitinase digests of a shark skin-derived chondroitin sulfate/dermatan sulfate hybrid chain (SS-CS/DS) (Native) by anion-exchange HPLC. SS-CS/DS (Native) was digested with chondroitinase ABC (A), chondroitinase B (B), or chondroitinase AC-I (C), and the digests were labeled with 2AB and analyzed by HPLC on a silica PA-03 column using a linear concentration gradient (dashed line) of NaH₂PO₄ from 16 to 530 mM over 60 minutes. The peaks obtained before 10 minutes are attributed to reagents. The elution peaks of unsaturated CS disaccharides labeled with 2AB are indicated by arrows as follows: 1, ΔHexUAα1-3GlcNAc; 2, ΔHexUAα1-3GalNAc (abbreviated to ΔDi-0S or ΔO); 3, ΔHexUAα1-3GalNAc(6S) (abbreviated to ΔDi-6S or ΔC); 4, ΔHexUAα1-3GalNAc(4S) (abbreviated to ΔDi-4S or ΔA); 5, ΔHexUA(2S) α1-3GalNAc(6S) (abbreviated to ΔDi-diS_{D} or ΔD); 6, ΔHexUA(2S) α1-3GalNAc(4S) (abbreviated to ΔDi-diS_{B} or AB); 7, ΔHexUA α1-3GalNAc(4S,6S) (abbreviated to ΔDi-diS_{E} or ΔE); 8, ΔHexUA(2S) α1-3GalNAc(4S,6S) (abbreviated to ΔDi-triS or ΔT);

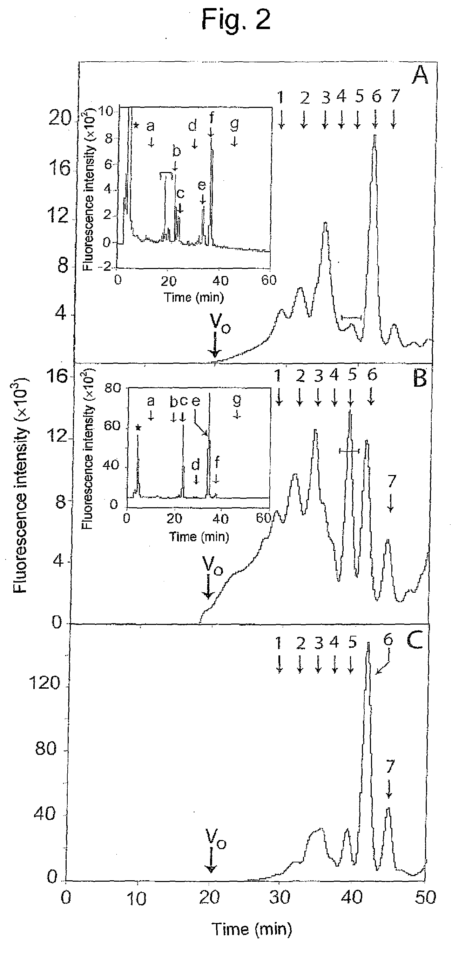

Figure 2 is a diagram showing a gel filtration analysis result of various chondroitinase digests of isolated SS-CS/DS (Native). SS-CS/DS (Native) (1 µg) was digested with chondroitinase AC-I (A) or chondroitinase B (B) or sequentially with chondroitinase B followed by chondroitinase AC-I (C), and the digests were labeled with 2AB and analyzed on a Superdex Peptide column. The elution positions of standard disaccharides and oligosaccharides are indicated by arrows as follows: 1, CS-decasaccharides; 2, CS-octasaccharides; 3, CS-hexasaccharides; 4, CS-tetrasaccharides; 5, disulfated CS-disaccharides; 6, monosulfated CS-disaccharides; 7, unsulfated CS-disaccharides. The elution positions of these oligosaccharides were previously measured (Bao, X et al., (2004) J. Biol. Chem. 79, 9765-9776). V₀ represents void volume, and the total volume (Vₜ) was 24 ml. Disulfated disaccharide fractions (indicated by bars) obtained from the chondroitinase AC-I (A) and chondroitinase B (B) digests were subjected to anion-exchange HPLC on a PA-03 column, and the results thereof are indicated by inserted panels A and B. The peaks represented by asterisks and brackets are attributed to reagents. The elution positions of disaccharides are indicated by arrows with alphabets as follows: a, ΔHexUAα1-3GalNAc (ΔO); b, ΔHexUAα1-3GalNAc(6S) (ΔC); c, ΔHexUAα1-3GalNAc(4S) (ΔA); d, ΔHexUA(2S) α1-3GalNAc(6S) (ΔD); e, ΔHexUA(2S) α1-3GalNAc(4S) (ΔB); f, ΔHexUAα1-3GalNAc(4S,6S) (ΔE); g, ΔHexUA(2S) α1-3GalNAc(4S,6S) (ΔT);
Figure 3 is a diagram showing a result of a gel filtration chromatography conducted on a Superdex-200 column on SS-CS/DS (Native) (◆), SS-CS/DS 1.0 M NaCl eluted fraction (1.0 M) (▲), and SS-CS/DS 1.5 M NaCl eluted fraction (1.5 M) (■) for determining a molecular size;
Figure 4 is a diagram showing the binding of various growth factors to SS-CS/DS (1.0 M and 1.5 M). Figure 4A shows a result of response to SS-CS/DS (1.0 M) (open bar) and SS-CS/DS (1.5 M) (solid bar). Figure 4B shows a result of response to heparin as a control experiment;
Figure 5-1 is a diagram showing a sensorgram of SS-CS/DS (1.5 M) binding depending on growth factor concentrations. The growth factors tested are FGF-2 (A) and FGF-10 (B);
Figure 5-2 is a diagram showing a sensorgram of SS-CS/DS (1.5 M) binding depending on growth factor concentrations. The growth factors tested are FGF-18 (C) and HB EGF (D);
Figure 5-3 is a diagram showing a sensorgram of SS-CS/DS (1.5 M) binding depending on growth factor concentrations. The growth factors tested are MK (E) and PTN (F);
Figure 6 is a diagram showing an analysis result of binding of SS-CS/DS (1.5 M) to BDNF or GDNF by use of BIAcore J System. Solid bars represent SS-CS/DS (1.5 M), and open bars represent a hagfish-derived chondroitin sulfate/dermatan sulfate hybrid chain (HN-CS/DS) (Figure 6A). Sensorgrams obtained with varying concentrations of BDNF and GDNF are shown in Figures 6B (BDNF) and 6C (GDNF). Arrows represent the respective beginnings of association and dissociation phases;

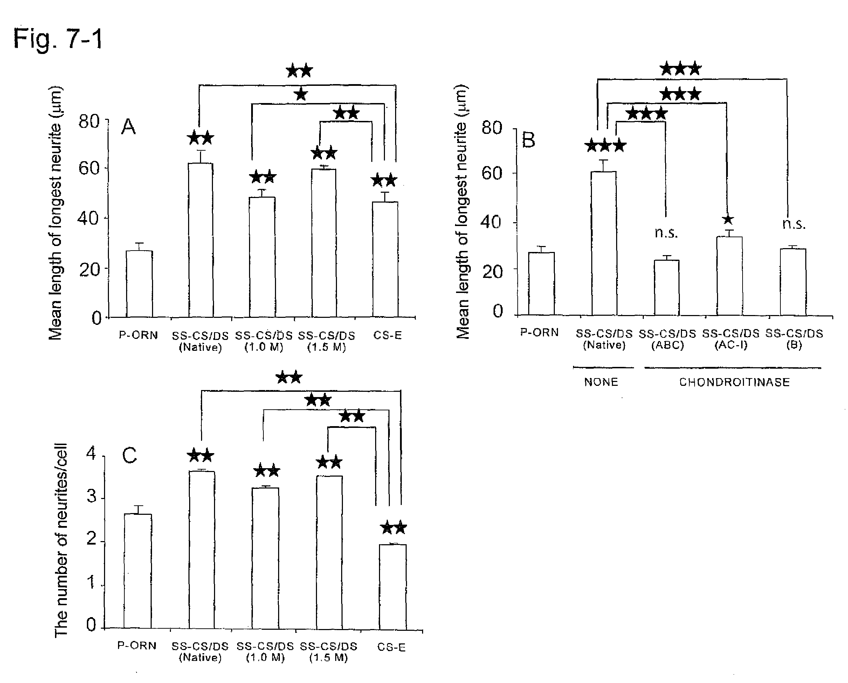

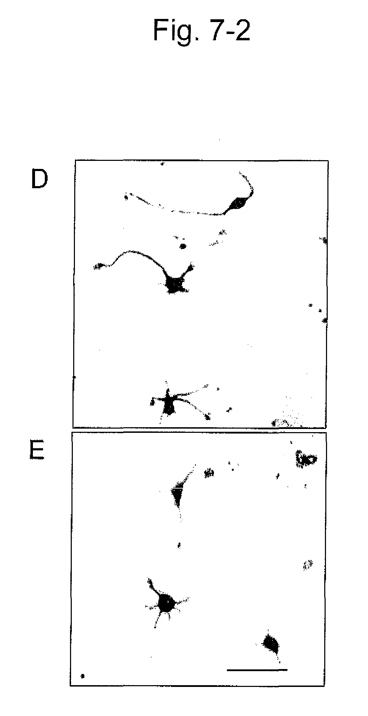

Figure 7-1 is a diagram showing the neurite outgrowth-promoting activity of three SS-CS/DS preparations (Native, 1.0 M, and 1.5 M). Three SS-CS/DS preparations (Native, 1.0 M, and 1.5 M) (0.67 µg as uronic acid) were applied onto coverslips precoated with P-ORN, on which primary cultured hippocampal neuronal cells derived from E16 mouse embryos were cultured in an Eagle's minimum essential medium for 24 hours. In Figure 7-1A, the mean length of the longest axonal process was measured for 100 randomly selected neurons cultured on three SS-CS/DS preparations (Native, 1.0 M, and 1.5 M). A squid cartilage-derived chondroitin sulfate chain (CS-E) and P-ORN alone were used as a positive control and negative control, respectively (★★: p<0.001, ★: p<0.005). Figure 7-1B shows a result of examination in the same way of SS-CS/DS (Native) digested with various chondroitinases (n.s.: no significant difference, ★★★: p<0.001, ★: p<0.05). Figure 7-1C is a graph of the number of neurites induced by the respective fractions of three SS-CS/DS preparations undigested (n.s.: no significant difference, ★★: p<0.001). Values obtained from two separate experiments are indicated as mean±SE;
Figure 7-2 is a photograph showing the neurite outgrowth-promoting activity of three SS-CS/DS preparations (Native, 1.0 M, and 1.5 M). Figure 7-2D shows the morphology of neurons cultured on SS-DS, and Figure 7-2E shows the morphology of neurons cultured on a coverslip coated with P-ORN alone;
Figure 8 is an anti-factor IIa activity measurement result of three SS-CS/DS preparations (Native, 1.0 M, and 1.5 M). Heparin (●) and pig skin-derived DS (■) were used as positive controls. The test was conducted on SS-CS/DS (Native) (◆), SS-CS/DS (1.0 M) (▲), and SS-CS/DS (1.5 M) (x);
Figure 9-1 shows a sensorgram of immobilized HN-CS/DS depending on growth factor concentrations. The growth factors tested are FGF-2 (A) and FGF-10 (B);
Figure 9-2 is a diagram showing a sensorgram of immobilized HN-CS/DS depending on growth factor concentrations. The growth factors tested are FGF-16 (C) and FGF-18 (D);
Figure 9-3 is a diagram showing a sensorgram of immobilized HN-CS/DS depending on growth factor concentrations. The growth factors tested are MK (E) and PTN (F);
Figure 9-4 is a diagram showing a sensorgram of immobilized HN-CS/DS depending on growth factor concentrations. The growth factors tested are HB-EGF (G) and VEGF (H);
Figure 10 is a diagram showing overlaid sensorgrams of varying concentrations of BDNF and GDNF perfused onto HN-CS/DS-immobilized sensor chips;
Figure 11-1 is a photograph showing the neurite outgrowth-promoting activity of digests of HN-CS/DS. Figure 11-1A shows neurons cultured on a coverslip coated with HN-CS/DS, and Figure 11-1B shows cells cultured on a coverslip coated with P-ORN alone; and
Figure 11-2 is a diagram showing the neurite outgrowth-promoting activity of digests of HN-CS/DS. Figures 11-2C and 11-2D show the length of neurites and the number of neurites, respectively.

### Best Mode for Carrying Out the Invention

The present invention relates to a chondroitin sulfate/dermatan sulfate hybrid chain and to glycosaminoglycan comprising the chain. The present invention further relates to a chondroitin sulfate/dermatan sulfate hybrid chain comprising a disaccharide GlcUA(2S)-GalNAc(4S) and to glycosaminoglycan comprising the chain. The glycosaminoglycan contains 60% or more, preferably 70% or more, more preferably 80% or more, even more preferably 90% or more, particularly preferably 95% or more (w/w, in all the cases), of the hybrid chain, and most preferably, substantially consists of the hybrid chain without other sugar chain components detected. However, the chondroitin sulfate/dermatan sulfate hybrid chain and the glycosaminoglycan comprising the chain of the present invention may contain 3% or less by weight, preferably 2% or less by weight, more preferably 1.2% or less by weight (e.g., approximately 0.6 to 1.2% by weight), of a peptide covalently linked to the sugar chain thereof

In the present invention, the chondroitin sulfate/dermatan sulfate hybrid chain refers to a polysaccharide chain with a disaccharide unit structure that contains both of the disaccharide unit of chondroitin sulfate and the disaccharide unit of dermatan sulfate. The types and contents of disaccharide units contained in the chondroitin sulfate/dermatan sulfate hybrid chain are not limited. For example, the following structure has been isolated from chondroitin sulfate:
G1cUAβ1-3GalNAc(6S) β1-4GlcUAβ1-3GalNAc(6S) β1-4GlcUAβ1-3GalNAc(6S) β1-4Glc UAβ1-3GalNAc(6S).

On the other hand, the following structure has been isolated from dermatan sulfate:
IdoUAα1-3GalNAc(4S) β1-4IdoUAα1-3GalNAc(4S) β1-4IdoUAα1-3GalNAc(4S) β1-4IdoU Aα1-3GalNAc(4S).

The hybrid type refers to a structure where IdoUA and GlcUA are present in the same sugar chain. Examples thereof include the following structures:
1)
   GlcUAβ1-3GalNAc(6S) β1-4IdoUAα1-3GalNAc(6S)β1-4GlcUAβ1-3GalNAc(6S) β1-4GlcU Aβ1-3GalNAc(6S),
2)
   GlcUAβ1-3GalNAc(6S) β1-4IdoUAα1-3GalNAc(4S) β1-4GlcUAβ1-3GalNAc(6S) β1-4Glc UAβ1-3GalNAc(6S), and
3)
   IdoUAα1-3GalNAc(4S) β1-4GlcUAβ1-3GalNAc(4S) β1-4IdoUAα1-3GalNAc(4S) β1-4IdoU Aα1-3GalNAc(4S).

The chondroitin sulfate/dermatan sulfate hybrid chain can be separated and purified from the bodies of fishes other than bony fishes. Examples of the fishes other than bony fishes include cartilaginous fishes and fishes belonging to cyclostomes. The bodies of fishes are not limited and can appropriately be selected according to the content of the chondroitin sulfate/dermatan sulfate hybrid chain or ease of separation/purification procedures depending on the types of the fishes. For example, shark skin and hagfish notochord can be used. Glycosaminoglycan fractions obtained from samples by acetone extraction, protease treatment, and trichloroacetic acid extraction are further subjected to the steps of CPC precipitation, hyaluronidase digestion, nitrous acid treatment, and desalting to obtain chondroitin sulfate/dermatan sulfate hybrid chain fractions. The finally obtained chondroitin sulfate/dermatan sulfate hybrid chain fractions can be further fractionated by applying them onto an anion-exchange resin, and eluting the chondroitin sulfate/dermatan sulfate hybrid chains adsorbed on the resin with a NaCl-containing eluent. Two chondroitin sulfate/dermatan sulfate hybrid chain fractions, a fraction eluted with approximately 1.0 M NaCl and a fraction eluted with approximately 1.5 M NaCl, are obtained. Both of the fractions are substantially pure chondroitin sulfate/dermatan sulfate hybrid chains. Those contained in both of the fractions have almost equal molecular weights. An average molecular weight thereof, for example an average molecular weight of a shark skin-derived chondroitin sulfate/dermatan sulfate hybrid chain, is approximately 65 to 75 kDa, preferably approximately 68 to 72 kDa, most preferably approximately 70 kDa, while an average molecular weight of a hagfish notochord-derived chondroitin sulfate/dermatan sulfate hybrid chain is approximately 18 kDa. The degree of sulfation per disaccharide molecule of the shark skin-derived chondroitin sulfate/dermatan sulfate hybrid chain of the present invention is less than 1.2, that is, falls within the range of preferably approximately 0.70 or more to less than 1.20, more preferably approximately 0.80 or more to less than approximately 1.20, particularly preferably approximately 0.87 to 1.17.

Each step of separation and purification of the chondroitin sulfate/dermatan sulfate hybrid chain from fishes other than bony fishes such as shark skin or hagfish notochord may be practiced by a method generally known by those skilled in the art. Embodiments of a series of steps are described below in Examples by way of illustration and however, are not limited to the described method.

In the present specification, the chondroitin sulfate/dermatan sulfate hybrid chain is also referred to as CS/DS. Moreover, prefixes SS and HN are added thereto to represent those derived from shark skin and hagfish notochord, respectively. For example, a fraction obtained from a shark skin sample by the hyaluronidase digestion, nitrous acid treatment, and desalting is referred to as SS-CS/DS (Native), and a fraction eluted with approximately 1.0 M NaCl and a fraction eluted with approximately 1.5 M NaCl, from the SS-CS/DS (Native) fraction further subjected to an anion-exchange resin are referred to as SS-CS/DS (1.0 M) and SS-CS/DS (1.5 M), respectively.

The composition analysis of these shark skin-derived chondroitin sulfate/dermatan sulfate hybrid chain fractions was conducted by digestion with chondroitinase ABC, B, or AC-I. As a result, all the SS-CS/DS fractions contained GlcUAβ1-3GalkNAc(4S) (A unit) or IdoUAα1-3GalNAc(4S) (iA unit) and GlcUAβ1-3GalNAc(6S) (C unit) or IdoUAα1-3GalNAc(6S) (iC unit) as main components. It was also shown that they contain all or some of disulfated disaccharides GlcUA(2S)β1-3GalNAc(6S) (D unit), IdoUA(2S)α1-3GalNAc(6S) (iD unit), GlcUA(2S)β1-3GalNAc(4S) (B unit), IdoUA(2S)α1-3GalNAc(4S) (iB unit), GlcUAβ1-3GalNAc(4S,6S) (E unit), and IdoUAα1-3GalNAc(4S,6S) (iE unit). Chondroitin sulfate and dermatan sulfate in these fractions are composed of quite diverse disaccharide molecules. Especially, a disaccharide molecule with a GlcUA(2S)-GalNAc(4S) structure was detected. The chondroitin sulfate/dermatan sulfate hybrid chain that contains the disaccharide molecule with the GlcUA(2S)-GalNAc(4S) structure was found for the first time by the present invention and is probably a property unique to the shark skin-derived chondroitin sulfate/dermatan sulfate hybrid chain.

As described above, the shark skin-derived chondroitin sulfate/dermatan sulfate hybrid chain must contain GlcUA(2S)-GalNAc(4S) (B unit) and further comprises at least one unit (i.e., 1, 2, 3, 4, or 4 units) selected from the group consisting of GlcUA-GalNAc(4S) (A unit), GlcUA-GalNAc(6S) (C unit), GlcUA(2S)-GalNAc(6S) (D unit), GlcUA-GalNAc(4S,6S) (E unit), and unsulfated GlcUA-GalNAc (O unit) and at least one unit (i.e., 1, 2, 3, 4, or 5 units) selected from the group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), and IdoUA-GalNAc(4S,6S) (iE unit). The hybrid chain of the present invention is a chondroitin sulfate/dermatan sulfate hybrid chain and as such, must simultaneously contain at least one member from among a chondroitin sulfate unit group consisting of GlcUA(2S)-GalNAc(4S) (B unit), GlcUA-GalNAc(4S) (A unit), GlcUA-GalNAc(6S) (C unit), GlcUA(2S)-GalNAc(6S) (D unit), GlcUA-GalNAc(4S,6S) (E unit), and unsulfated GlcUA-GalNAc (O unit) and at least one member from among a dermatan sulfate unit group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), and IdoUA-GalNAc(4S,6S) (iE unit).

As a result of composition analysis of the hagfish notochord-derived chondroitin sulfate/dermatan sulfate hybrid chain, the main components thereof were GlcUAβ1-3GalNAc(4S,6S) (E unit) or IdoUAα1-3GalNAc(4S,6S) (iE unit) and GlcUAβ1-3GalNAc(4S) (A unit) or IdoUAα1-3GalNAc(4S) (iA unit). It also contained all or some of GlcUAβ1-3GalNAc (O unit), GlcUAβ1-3GalNAc(6S) (C unit), IdoUAα1-3GalNAc(6S) (iC unit), GlcUA(2S)β1-3GalNAc(4S,6S) (T unit), and IdoUA(2S)α1-3GalNAc(4S,6S) (iT unit). Namely, the hagfish notochord-derived chondroitin sulfate/dermatan sulfate hybrid chain comprises at least one unit (i.e., 1, 2, 3, 4, or 5 units) selected from the group consisting of GlcUAβ1-3GalNAc(4S) (A unit), GlcUAβ1-3GalNAc(6S) (C unit), GlcUAβ1-3GalNAc(4S,6S) (E unit), GlcUA(2S) β1-3GalNAc(4S,6S) (T unit), and unsulfated GlcUA-GalNAc (O unit) and at least one unit (i.e., 1, 2, 3, or 4 units) selected from the group consisting of IdoUAα1-3GalNAc(4S) (iA unit), IdoUAα1-3GalNAc(6S) (iC unit), IdoUAα1-3GalNAc(4S,6S) (iE unit), and IdoUA(2S) α1-3GalNAc(4S,6S) (iT unit).

As described above, the chondroitin sulfate/dermatan sulfate hybrid chain derived from fishes except for bony fishes including shark and hagfish comprises at least one unit selected from the group consisting of GlcUA-GalNAc(4S) (A unit), GlcUA(2S)-GalNAc(4S) (B unit), GlcUA-GalNAc(6S) (C unit), GlcUA(2S)-GalNAc(6S) (D unit), GlcUA-GalNAc(4S,6S) (E unit), GlcUA(2S) β1-3GalNAc(4S,6S) (T unit), and unsulfated GlcUA-GalNAc (O unit) and at least one unit selected from the group consisting of IdoUAα1-3GalNAc(4S) (iA unit), IdoUA(2S)-GalNAc(4S) (iB unit), IdoUAα1-3GaNAc(6S) (iC unit), IdoUA(2S)-GalNAc(6S) (iD unit), IdoUA-GalNAc(4S,6S) (iE unit), and IdoUA(2S) α1-3GalNAc(4S,6S) (iT unit).

The chondroitin sulfate/dermatan sulfate hybrid chain of the present invention bonded to a variety of growth factors (fibroblast growth factor (FGF)-2, FGF-10, FGF-18, heparin-binding epidermal cell growth factor (HB-EGF), midkine (MK), pleiotrophin (PTN), brain-derived neurotrophic factor (BDNF), and glial cell-derived neurotrophic factor (GDNF)) with high affinity.

Furthermore, the chondroitin sulfate/dermatan sulfate hybrid chain of the present invention exhibits neurite outgrowth-promoting activity and anticoagulant activity.

The chondroitin sulfate/dermatan sulfate hybrid chain derived from fishes other than bony fishes such as shark skin or hagfish notochord is useful as therapeutic or preventive agent for neurodegenerative disease and for the promotion of nervous system development by virtue of such properties. The hybrid chain is also useful as an anticoagulant.

Thus, the present invention also encompasses a growth factor (FGF-2, FGF-10, FGF-18, HB-EGF, MK, PTN, BDNF, and GDNF)-binding agent, neurite outgrowth-promoting agent, and anticoagulant comprising glycosaminoglycan consisting of the chondroitin sulfate/dermatan sulfate hybrid chain derived from fishes other than bony fishes such as the chondroitin sulfate/dermatan sulfate hybrid chains contained in the shark skin- and hagfish notochord-derived fractions. Those skilled in the art can easily accomplish production methods of these agents. For example, glycosaminoglycan containing the chondroitin sulfate/derinatan sulfate hybrid chain is completely or partially purified from shark skin as described above, and the obtained fractions may be mixed, if necessary, with appropriate carriers and/or solvents.

The glycosaminoglycan is available as a pharmaceutical composition.

Particularly, the glycosaminoglycan is available as a pharmaceutical composition for neurodegenerative disease prevention or treatment by virtue of its neurite outgrowth-promoting activity. Since neurodegenerative diseases are attended with the denaturation and dropout of neurites in neurons, the glycosaminoglycan is capable of preventing these events or promoting the outgrowth of new neurites and as such, is capable of alleviating the progression of symptoms of the diseases or the symptoms.

For these preparations or pharmaceutical compositions, it is preferred that the glycosaminoglycan should finally act at a concentration of 1 to 20 µg/ml, preferably 1 to 10 µg/ml, more preferably 1 to 5 µg/ml.

For example, when the glycosaminoglycan containing the chondroitin sulfate/dermatan sulfate hybrid chain or a fraction containing the glycosaminoglycan is applied to a neurite outgrowth-promoting agent, fractions obtained by completely or partially purifying the glycosaminoglycan containing the chondroitin sulfate/dermatan sulfate hybrid chain from shark skin as described above can be administered in a way capable of bringing it in contact with neurons of target individuals in need of promotion of neurite outgrowth. An appropriate administration method can be selected by those skilled in the art. The agent may be administered locally so that it is capable of being delivered to neurons desired to elongate neurites or to the neighborhoods of the neurons. It is preferred that the agent should be delivered at a concentration on the order of 1 to 10 µg/ml, more preferably 1 to 5 µg/ml, even more preferably 2 µg/ml. The same holds true for the glycosaminoglycan containing the chondroitin sulfate/dermatan sulfate hybrid chain derived from other fishes except for bony fishes such as hagfish notochord.

For example, when the glycosaminoglycan or a fraction containing the glycosaminoglycan is applied to a growth factor-binding agent, the glycosaminoglycan or the fraction containing the glycosaminoglycan in an amount appropriate for the purpose (which differs depending on target growth factors) may be mixed and contacted with growth factors.

Furthermore, when the glycosaminoglycan or a fraction containing the glycosaminoglycan is applied to an anticoagulant, the glycosaminoglycan or the fraction containing the glycosaminoglycan in an amount appropriate for the purpose may be mixed and contacted with growth factors.

The glycosaminoglycan at a concentration of 0.1 µg or more, preferably 1 to 10 µg/ml, is mixed and contacted with blood and thereby exerts anticoagulant activity.

Target individuals to which these agents are applied may be arbitrary mammals.

Separation methods of the chondroitin sulfate/dermatan sulfate hybrid chain fractions derived from fishes other than bony fishes of the present invention and the analysis of their properties will be described in detail in Examples below by taking shark skin and hagfish notochord as examples. However, the present invention is not intended to be limited to the descriptions of Examples.

### Example 1 Separation of chondroitin sulfate/dermatan sulfate hybrid chain fractions from shark skin

### <Procedures>

### Isolation of glycosaminoglycan

*Prionace glauca* skin (dry weight 85 g) was delipidated three times by acetone extraction and completely air-dried. The resulting product was suspended in water and then held in boiling water for 30 minutes to inactivate protease.

The suspension was supplemented with borate-NaOH buffer and calcium chloride at the final concentrations of 0.1 M and 10 mM, respectively, and digested With protease (actinase; 2% by weight with respect to that of the sample) at 60°C for 24 hours. After 24 hours and 48 hours, actinase (1% by weight with respect to that of the sample) was freshly added thereto to perform digestion. Then, 50% trichloroacetic acid was added at the final concentration of 5%, followed by centrifugation to precipitate proteins. The pellet was resuspended in 5% trichloroacetic acid, followed by additional centrifugation to collect the supernatants.

Excessive trichloroacetic acid in the supernatant was removed by diethyl ether extraction. Glycosaminoglycan was precipitated by adding thereto 4 volumes of 80% ethanol containing 5% sodium acetate and leaving it overnight at 4°C, and collected by further centrifugation, followed by drying.

### Purification of glycosaminoglycan

The glycosaminoglycan-containing precipitate was solubilized with 0.02 M Na₂SO₄, then supplemented with 10% (w/v) cetylpyridinium chloride (CPC)/0.02 M Na₂SO₄, and left overnight at room temperature. The obtained flocculent precipitate was redissolved with 100:15 (v/v) 2M NaCl/ethanol solution and precipitated again by adding 3 volumes of 99.5% ethanol. The step described above was repeated three times, and precipitation in water and drying were finally performed. An additional CPC precipitation step was practiced in the same way by keeping a critical electrolyte concentration at 0.5 M NaCl, to perform the removal of hyaluronic acid and the enrichment of glycosaminoglycan.

### Hyaluronidase digestion

The CPC precipitate thus obtained was dissolved in 0.02 M acetate buffer (pH 6) containing 0.15 M NaCl and digested with shaking at 60°C with 50 TRU (turbidity reducing units) of *Streptomyces* hyaluronidase for 5 hours. It was further digested for 12 hours by further adding 50 TRU of hyaluronidase. After cellulose acetate membrane electrophoresis to confirm the digestion of the hyaluronic acid, the digest was treated with TCA to remove proteins, and supplemented with ethanol at a concentration of 64% to precipitate glycosaminoglycan.

### Nitrous acid treatment

The precipitate obtained after hyaluronidase digestion was treated by standing at room temperature for 40 minutes with nitrous acid obtained by mixing equal volumes of 0.5 mmole of sulfuric acid and 0.5 mmole of barium nitrite. An aliquot of freshly prepared nitrous acid was added again thereto and further left undisturbed for 40 minutes. The treated sample was neutralized with 0.5 M Na₂CO₃ and desalted with a Sephadex G-50 column (1x56 cm) (eluent: 0.2 M ammonium bicarbonate, flow rate: 0.6 ml/min). The elution was monitored at 210 nm, and eluted fractions were collected and subjected to repetitive procedures of freeze-drying and reconstitution in water.

### Purification of chondroitin sulfate/dermatan sulfate hybrid chain with C18 and anion-exchange columns

The desalted glycosaminoglycan was passed through a Sep-Pak C18 cartridge column and eluted with water and subsequently with 100% methanol. In this context, the eluted chondroitin sulfate/dermatan sulfate hybrid chain without free peptides is referred to as SS-CS/DS (Native). This preparation contains a slight amount (approximately 0.6 to 1.2% by weight) of peptides covalently linked to the sugar chain.

SS-CS/DS (Native) was passed through an anion-exchange cartridge and eluted stepwise with 300 mM phosphate buffer containing 0.15, 0.5, 1.0, 1.5, or 2.0 M NaCl, followed by desalting on a PD-10 column with 50 mM pyridine acetate buffer (pH 5.0) as an eluent.

### <Result>

As a result of purification, fractions eluted with the buffers containing 1.0 M and 1.5 M NaCl contained 20% and 80% chondroitin sulfate/dermatan sulfate hybrid chains, respectively, which were in turn referred to as SS-CS/DS (1.0 M) and SS-CS/DS (1.5 M). Yields by purification are shown in Table 1.

**Table 1 Yields of SS-CS/DS**

| Yields of SS-CS/DS | | |
|---|---|---|
| Fraction | Yield g (%) | Uronic acid content (%) |
| Ethanol precipitation | 3.5 (4.2) | 12 |
| CPC precipitation (first) | 1.6(1.9) | 21 |
| CPC precipitation (second) | 0.4 (0.5) | 53 |
| Hyaluronidase digestion | 0.17 (0.2) | 39 |
| Nitrous acid treatment | 0.044 (0.05) | 33 |
| Anion-exchange chromatography | 0.036 (0.04) | 33 |
| 1.0 M eluted fraction | 0.007 (0.008) | 33 |
| 1.5 M eluted fraction | 0.029 (0.034) | 33 |

### Example 2 Disaccharide composition analysis

### <Procedures>

### Enzymatic digestion

Disaccharide composition analysis was conducted by analyzing a chondroitinase ABC, B, or AC-I digest.

Three SS-CS/DS preparations (Native, 1.0 M, and 1.5 M) (1 µg or 3.6 µg each) were digested with either of 10 mIU of chondroitinase ABC, 5 mIU of chondroitinase AC-I, or 2 mIU chondroitinase B, and the respective digests were labeled with 2-aminobenzamide (2AB) according to the previous report (Kinoshita, A., Sugahara, K (1999) Anal. Biochem. 269, 367-378) (excessive 2AB was removed with a water chloroform (1:1, v/v) mixture).

The disaccharides labeled with 2AB were diluted with 400 µl of 16 mM NaH₂PO₄ and analyzed by anion-exchange HPLC (PA-03 silica column using 16 mM and 530 mM NaH₂PO₄ solvents).

Alternatively, three SS-CS/DS preparations (Native, 1.0 M, and 1.5 M) (1.6 µg each) were digested with 10 mIU of chondroitinase ABC (total volume 10 µl), and 1/3 aliquots of the respective digests were digested with 10 mIU of chondro-4- or chondro-6-sulfatase. These digests were labeled with 2AB and analyzed by HPLC in the same way as above.

### Gel filtration analysis of chondroitinase digests of SS-DS on Superdex Peptide column

SS-CS/DS (Native) (1.0 µg) was digested with chondroitinase AC-I or B.

The chondroitinase AC-I digest and the chondroitinase B digest were then digested with chondroitinase B and chondroitinase AC-I, respectively. All the digests were labeled with 2AB, then dissolved in 0.2 M ammonium bicarbonate containing 7% 1-propanol, and analyzed on a Superdex Peptide column by fluorescence detection (flow rate: 0.4 ml/min).

### <Result>

### Composition analysis by chondroitinase ABC digestion

A result of analysis of three SS-CS/DS preparations (Native, 1.0 M, and 1.5 M) enzymatically digested is shown in Table 2.

All of these three preparations SS-CS/DS (Native), SS-CS/DS (1.0 M), and SS-CS/DS (1.5 M) were shown to contain ΔHexUAα1-3GalNAc (ΔDi-0S), ΔHexUAα1-3GalNAc(6S) (ΔDi-6S), ΔHexUAα1-3GlNAc(4S) (ΔDi-4S), ΔDi-diS_{D}, ΔDi-diS_{B}, and ΔDi-diS_{E} at various ratios.

The main components of the three SS-CS/DS preparations (Native, 1.0 M, and 1.5 M) were ΔDi-4S and ΔDi-6S, and the ratios of ΔDi-4S and ΔDi-6S to all the disaccharides of SS-CS/DS (Native), SS-CS/DS (1.0 M), and SS-CS/DS (1.5 M) were 48.9% and 21.3%, 43.5% and 21.3%, and 55.0% and 16.6%, respectively (Table 2). The ratio of ΔDi-0S in SS-CS/DS (1.0 M) was 23.8%, which was higher than that in SS-CS/DS (Native) (10.5%) and SS-CS/DS (1.5 M) (5.8%). By contrast, the ratio of disulfated disaccharides (ΔDi-diS_{D}, ΔDi-diS_{B}, and ΔDi-diS_{E}) in SS-CS/DS (1.5 M) was 22.6%, which was higher than that in SS-CS/DS (Native) (19.3%) and SS-CS/DS (1.0 M) (11.4%).

These results demonstrated that SS-CS/DS (Native) contains various kinds of CS/DS chains with varying degrees of sulfation.

Most of marine organism-derived chondroitin sulfate and dermatan sulfate preparations isolated so far including those from shark cartilage, squid cartilage, ascidians, sea urchin, and hagfish were highly sulfated products (sulfate to disaccharide molar ratio (S/Unit)=1.2 to 1.9), which are largely made up of disulfated disaccharides "E" unit, "iE" unit, "D" unit, "iD" unit, or "iB" (IdoUA(2S)-GalNAc(4S)) unit. The three SS-CS/DS preparations (Native, 1.0 M, and 1.5 M) of the present invention are unique in that despite of their S/Unit as low as 0.87 to 1.17 (Table 2), they contain a considerable amount of several disulfated disaccharide units and contain even a unsulfated disaccharide unit (O unit).

### Composition analysis by chondroitinase B digestion

The three SS-CS/DS preparations (Native, 1.0 M, and 1.5 M) generated ΔDi-4S and ΔDi-diS_{B} as a result of chondroitinase B digestion (Table 2). The total amount of disaccharides obtained by chondroitinase B digestion was only on the order of 2% of the total amount of disaccharides obtained by chondroitinase ABC digestion.

This liberation of slight amounts of ΔDi-4S and ΔDi-diS_{B} by chondroitinase B treatment suggests that most of their original units are present as GlcUA-GalNAc(4S) (A unit) or GlcUA(2S)-GalNAc(4S) (B unit) rather than IdoUA-GalNAc(4S) (iA unit) or IdoUA(2S)-GalNAc(4S) (iB unit). It is also considered that the great majority of iA units and iB units do not form clusters and therefore possess resistance to chondroitinase B digestion.

Chondroitinase B digestion did not liberate ΔDi-6S, which was however detected to some extent after chondroitinase AC-I digestion, suggesting that the 6-0-sulfated units are present as GlcUA-GalNAc(6S) (C unit) or IdoUA-GalNAc(6S) (iC unit). They are likely to have resistance to the enzyme or to be contained in sequences resistant to the enzyme.

### Composition analysis by chondroitinase AC-I digestion

In the chondroitinase AC-I digestion of the three SS-CS/DS preparations (Native, 1.0 M, and 1.5 M), disulfated disaccharides were hardly detected, while nonsulfated and monosulfated disaccharides including ΔDi-0S, ΔDi-6S, and ΔDi-4S, which accounted for only 10% of the total disaccharide amounts of the SS-CS/DS preparations were detected (Table 2). This result suggests that at least some of 4-O-sulfated units and 6-O-sulfated units are present as "A" and "C" units.

### Gel filtration analysis of chondroitinase AC-I and B digests

To examine the distribution of IdoUA-containing units and GlcUA-containing units, SS-CS/DS (Native) (1.0 µg) was digested with chondroitinase AC-I or B, and their respective digests were analyzed by gel filtration chromatography on a Superdex Peptide column. Oligosaccharides (hexa-, octa-, and decasaccharides) were observed in both digests. This suggests that the GlcUA-containing units and IdoUA-containing units coexist in the polysaccharide chain and form a CS/DS hybrid structure (Figures 2A and 2B).

The chondroitinase AC-I and B digests mainly generated, as a result of digestion with chondroitinase B and AC-I, respectively, disaccharides except for digestion-resistant fragments corresponding to tetra- and hexasaccharides. This suggests that oligosaccharides having adjacent IdoUA-containing disaccharide units or GlcUA-containing disaccharide units are present (Figure 2C).

A smaller number of decasaccharides or larger oligosaccharides were present in the chondroitinase AC-I digest (Figure 2A) than the chondroitinase B digest (Figure 2B). This result supported a somewhat higher ratio of GlcUA than IdoUA.

The disaccharides (arrows 5, 6, and 7 in Figures 2A and 2B) obtained from either chondroitinase digestion may indicate the presence of adjacent sequences composed of GlcUA-containing units (10%) and IdoUA-containing units (2%). According to anion-exchange chromatography, the fraction corresponding to disulfated disaccharides of the chondroitinase AC-I digest showed the presence of ΔDi-diS_{E} as a major component and ΔDi-diS_{B} as a minor component, whereas disulfated disaccharides of the chondroitinase B digest showed the presence of ΔDi-diS_{B} as a main component and ΔDi-diS_{E} as a minor component. Disulfated disaccharides were not detected in the chondroitinase AC-I digest of SS-CS/DS (Native) by anion-exchange HPLC (Figure 1C), probably due to their almost undetectable amounts compared with other disaccharides (Table 2). However, the disulfated disaccharides of SS-CS/DS (Native) were detected when fractionated first on a Superdex Peptide column and then on an anion-exchange column. These results demonstrate that ΔDi-diS_{B} and ΔDi-diS_{E} in the SS-CS/DS preparations are derived from the GlcUA-containing disaccharide unit and IdoUA-containing disaccharide unit, that is, GlcUA(2S)/IdoUA(2S)-GalNAc(4S) and GlcUA/IdoUA-GalNAc(4S,6S). "B" unit containing GlcUA (GlcUA(2S)-GalNAc(4S)) was detected for the first time by this experiment. Although the values of these units shown in Table 2 are quite small, they represent only cleavable units, and additional B units are highly likely to be contained with high frequency. This B unit may be involved in the function of SS-CS/DS.

By contrast, ΔDi-diS_{D} was not detected in the chondroitinase AC-I or B digest (Figures 1B and 1C, Table 2). This shows that sequences containing D [GlcUA(2S)-GalNAc(6S)] or iD [IdoUA(2S)-GalNAc(6S)] unit are indigestible by chondroitinase AC-I or B, as previously reported (Yoshida, K. et al., (1993) Dermatan sulfate Proteoglycans Chemistry, Biology and Chemical Pathology (Scott, J. E., Ed.) pp 55-80, Portland Press). "D" and/or "iD" units were not detected by chondroitinase AC-II digestion, in spite of the fact that they exist at a considerable ratio (3%) and that chondroitinase AC-II efficiently dissociates D unit from CS oligosaccharide (Nadanaka, S. et al., (1998) J. Biol. Chem., 273, 33728-33734). This may be because ΔDi-diS_{D} generated by chondroitinase ABC is derived from iD unit, which possesses sensitivity to chondroitinase ABC but no sensitivity to chondroitinase B. However, the actual proportions of iD and D units in the three SS-CS/DS preparations are still unclear.

**Table 2 Disaccharide composition of SS-CS/DS preparations**

| Disaccharide composition | | | | | | | | | |
|---|---|---|---|---|---|---|---|---|---|
| | SS-CS/DS(Native) | | | SS-CS/DS(1.0 M) | | | SS-CS/DS(1.5M) | | |
| | ABC | B | AC-I | ABC | B | AC-I | ABC | B | AC-I |
| | pmoles (mole%) | | | | | | | | |
| ΔDi-0S | 31.5 (10.5) | - | 2.5 (8.3) | 71.5 (23.8) | - | 4.2 (14.0) | 17.4 (5.8) | - | 1.3 (4.3) |
| ΔDi-6S | 64.0 (21.3) | ND | 15.0 (50.0) | 63.9 (21.3) | ND | 9.1 (30.3) | 49.9 (16.6) | ND | 14.7 (49.0) |
| ΔDi-4S | 146.5 (48.9) | 3.3 (55.0) | 12.5 (14.7) | 130.5 (43.5) | 4.2 (70.0) | 16.7 (55.7) | 165.0 (55.0) | 3.4 (56.7) | 14.0 (46.7) |
| ΔDi-diS_{D} | 9.0 (3.0) | ND | ND | 6.3 (2.1) | ND | ND | 10.0 (3.4) | ND | ND |
| ΔDi-diS_{B} | 17.7 (5.9) | 2.7 (45.0) | 0.12 (0.04) | 8.0 (2.7) | 1.8 (30.0) | ND | 24.1 (8.0) | 2.6 (43.3) | ND |
| ΔDi-diS_{E} | 31.3 (10.4) | 0.39 (0.13) | 0.69 (0.23) | 19.8 (6.6) | ND | ND | 33.6 (11.2) | ND | ND |
| Total | 300 | 6.39 | 30.8 | 300 | 6.0 | 30.0 | 300 | 6.0 | 30.0 |
| S/Unit | 1.08 | - | - | 0.87 | - | - | 1.17 | - | - |

### Example 3 Molecular weight determination

### <Procedures>

The molecular sizes of the chondroitin sulfate/dermatan sulfate hybrid chains were measured by gel filtration using a Superdex 200 column (10×300 mm). Three SS-CS/DS preparations (Native, 1.0 M, and 1.5 M) (40 µg each) were loaded onto the column and eluted with 0.2 M ammonium acetate at a flow rate of 0.3 ml/min. The fractions were collected at 3-minute intervals, then evaporated to dryness, and dissolved with 100 µl of water. The measurement was performed according to the previous report (Chandrasekhar, S., et al (1987) Anal Biochem. 161, 103-108).

### <Result>

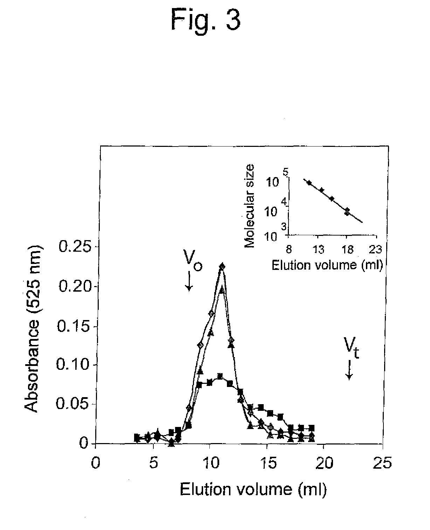

When the molecules of SS-CS/DS were examined by gel filtration column chromatography, all of the SS-CS/DS (Native), SS-CS/DS (1.0 M), and SS-CS/DS (1.5 M) preparations exhibited an average molecular weight of 70 kDa (Figure 3).

This molecular weight was much larger than those of the respective CS/DS chains derived from hagfish notochord (18 kDa), pig skin (19 kDa), eel skin (14 kDa), endothelial cells (30 kDa), and pig brain (40 kDa). Since chondroitin sulfate C chains from shark cartilage have a considerably large molecular weight (43 to 70 kDa), the molecular weight of the SS-CS/DS chain of the present invention may be characteristic of shark.

### Example 4 Interaction analysis of SS-CS/DS preparations (1.0 M and 1.5 M) with a variety of growth factors

### <Procedures>

The interaction analysis of SS-CS/DS preparations (1.0 M and 1.5 M) with a variety of growth factors (FGF-1, FGF-2, FGF-10, FGF-16, HB-EGF, PTN, and MK) was conducted with Interaction Analysis System (IAsys; Affinity sensors, Cambridge, UK) according to the previous report (Deepa, S. S., et. al. (2002) J. Biol. Chem. 277, 43707-43716).

The interaction was evaluated by determining affinity parameters dissociation constant k_{d} and association constant kₐ with FASTfit software (Affinity sensors, Cambridge, UK) to calculate a dissociation equilibrium constant (K_{d}=dissociation constant k_{d}/association constant kₐ).

### <Result>

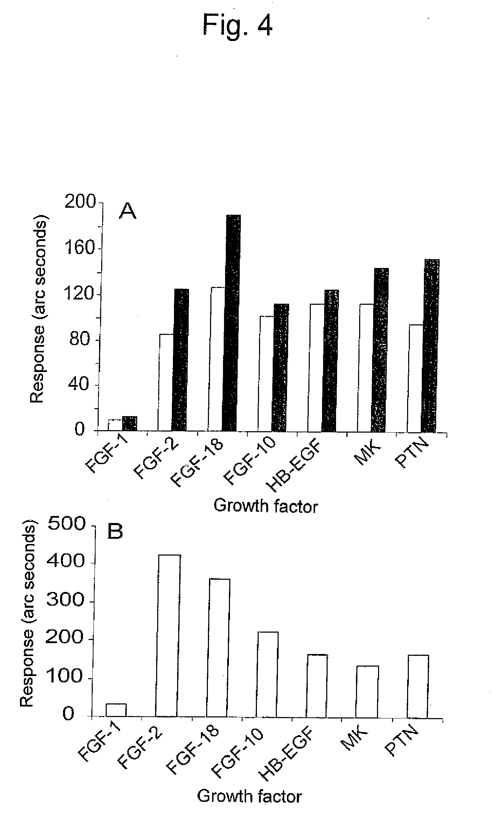

As a result of test by perfusing an excessive amount (1,500 to 3,500-fold) of each growth factor and bringing it in contact with the SS-CS/DS preparations (1.0 M and 1.5 M), all of them exhibited high interaction responses with all the growth factors except for FGF-1, though the responses differed depending on the growth factors. The degree of binding also differed depending on the SS-CS/DS preparations (1.0 M and 1.5 M), and the SS-CS/DS (1.5 M) preparation induced higher responsiveness than that of the SS-CS/DS (1.0 M) preparation (Figure 4).

Among the various growth factors tested, FGF-18 exhibited the highest response (180 arc seconds) to 5.0 fmols of immobilized SS-CS/DS (1.5 M), which was comparable to 1.2 ng (43 fmol) of the growth factor. This shows that one SS-CS/DS chain bonded to 8.6 molecules of FGF-18 on average. Likewise, the maximum amount of binding of each growth factor to SS-CS/DS (1.5 M) was calculated to evaluate a binding capacity for all the growth factors. The result thereof is summarized in Table 3 in comparison with the previously reported binding capacities of CS-H (hagfish-derived chondroitin sulfate/dermatan sulfate hybrid chain) and heparin (Hep).

**Table 3 Binding capacity of SS-CS/DS (1.5 M) preparation to a variety of growth factors**

| Growth factor | Average molecular weight | Molar ratio of binding | | | Binding capacity | | |
|---|---|---|---|---|---|---|---|
| | | SS-CS/DS (1.5 M) | CS-H | Hep | SS-CS/DS (1.5 M) | CS-H | Hep |
| | (kDa) | (mole/mole) | | | (mole/30 disaccharides) | | |
| FGF-2 | 17.5 | 9.6 | 0.5 | 9 | 2.1 | 0.5 | 11.7 |
| FGF-10 | 24.0 | 6.2 | 0.2 | 5 | 1.4 | 0.2 | 6.5 |
| FGF-12 | 28.0 | 8.6 | 1.3 | 5 | 1.9 | 1.2 | 6.5 |
| HB-EGF | 12.0 | 13.8 | 0.7 | 5 | 3.1 | 0.6 | 6.5 |
| MK | 13.4 | 14.4 | 0.6 | 4 | 3.2 | 0.5 | 5.2 |
| PTN | 15.4 | 13.2 | 1.0 | 4 | 2.9 | 0.9 | 5.2 |

These results demonstrated that the SS-CS/DS chains bind to a larger number of Hep-binding growth factors than CS-H chains do. SS-CS/DS (1.5 M), even when compared with Hep, bonded to a larger number of most of the growth factors per polysaccharide chain.

When a binding capacity is expressed as the number of growth factors binding to the glycosaminoglycan chain of the same length, SS-CS/DS (1.5 M) also had a binding capacity several-fold higher than that of CS-H (right column in Table 3). Considering that the degree of sulfation (S/Unit=1.17) of SS-CS/DS (1.5 M) is lower than that of Hep (S/Unit=2.55) despite its binding capacity several-fold lower than that of Hep, the sequence specificity is probably shown to be high.

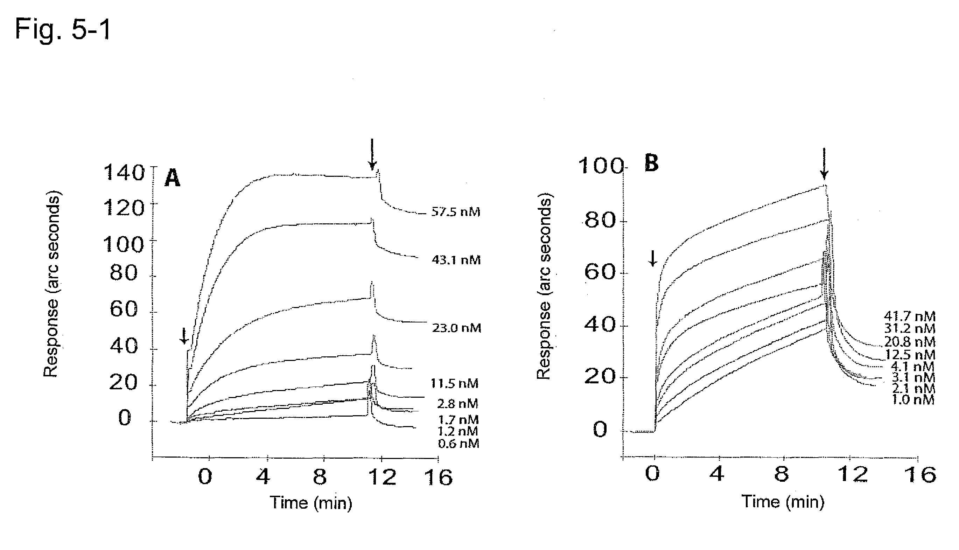

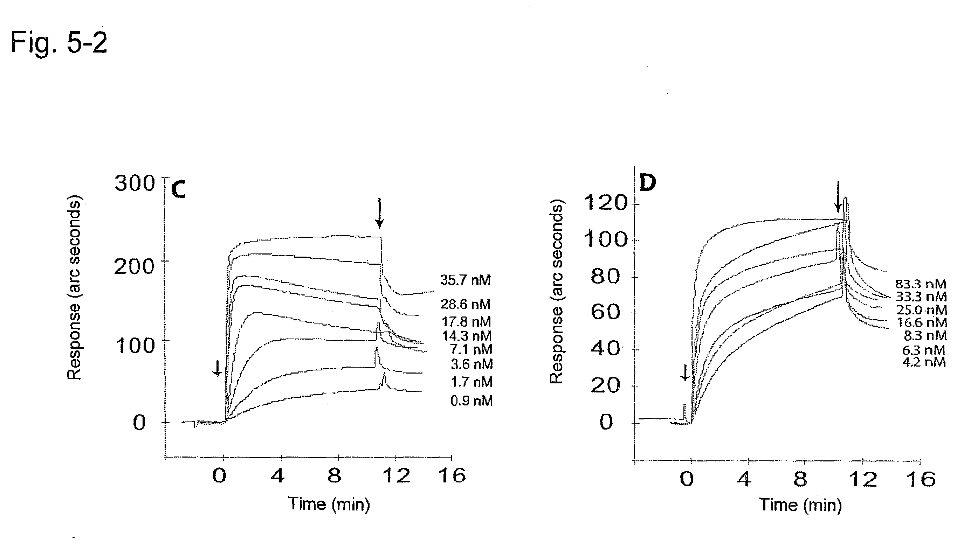

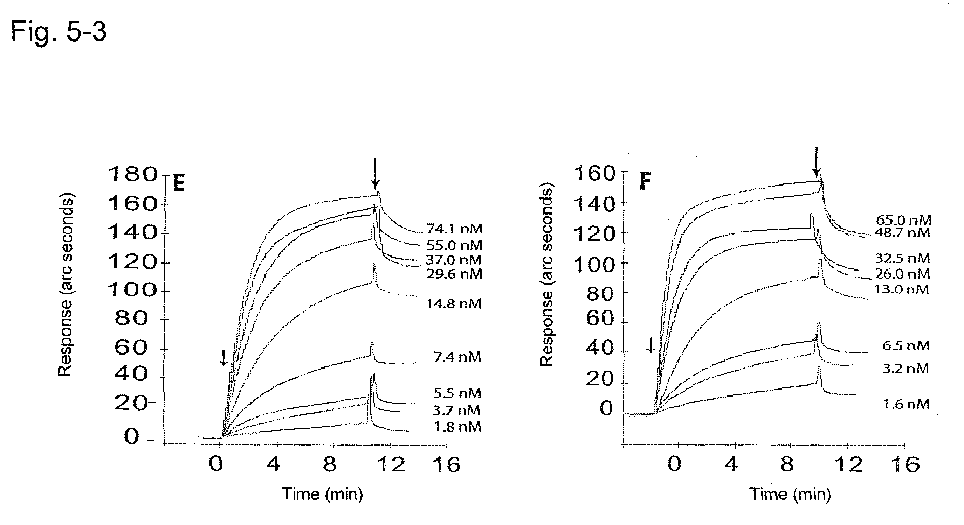

To determine binding affinity, varying concentrations of growth factors were allowed to interact with the immobilized SS-CS/DS (1.5 M) preparation. Sensorgrams of SS-CS/DS (1.5 M) binding to FGF-2 (A), FGF-10 (B), FGF-18 (C), HB-EGF (D), MK (E), and PTN (F) are shown in Figures 5-1 to 5-3. All the growth factors exhibited binding patterns typical of growth factors.

K_{d} obtained by the IAsys system revealed that all the growth factors have high affinity to SS-CS/DS (1.5 M) (Table 4). The highest affinity was exhibited in FGF-18 (K_{d}=4.4 nM) and HB-EGF (K_{d}=4.5 nM), followed by PTN (K_{d}=7.6 nM) and FGF-10 (K_{d}=8.6 nM).

The interaction between PTN and DS (derived from pig skin; containing only IdoUA) has already been reported, with K_{d} of 51 nM obtained by the IAsys system (Vacherot, F et al., (1999) J. Biol. Chem. 274, 7741-7747), whereas K_{d} for SS-CS/DS (1.5 M) was 7.6 nM (Table 4).

**Table 4**

| Growth factor | kₐ (M⁻¹S⁻¹) | K_{d} (S⁻¹) | r | K_{d} (nM) |
|---|---|---|---|---|
| SS-CS/DS (1.5 M) | | | | |
| FGF-2 | (2.2±1.5) × 10⁵ | (0.1±0.04) × 10⁻² | 0.980 | 13.8±11.1 |
| FGF-10 | (1.9±0.2) × 10⁵ | (0.2±0.03) × 10⁻² | 0.980 | 8.6±2.3 |
| FGF-18 | (31.3±4.8) × 10⁵ | (1.2±0.90) ×10⁻² | 0.995 | 4.4±3.6 |
| HB-EGF | (4.7±0.6) × 10⁵ | (0.2±0.10) × 10⁻² | 0.963 | 4.5±2.5 |
| MK | (1.0±0.8) × 10⁵ | (0.2±0.04) × 10⁻² | 0.972 | 58.5±21.5 |
| PTN | (2.9±1.5) × 10⁵ | (0.2±0.09) × 10⁻² | 0.990 | 7.6±5.5 |

| Hep | | | | |
|---|---|---|---|---|
| FGF-2 | (2.4±0.2) × 10⁵ | (0.2±0.06) × 10⁻² | 0.991 | 8.6±3.2 |
| FGF-10 | (1.4±0.3) × 10⁵ | (0.2±0.15) ×10⁻² | 0.966 | 17.4±14.4 |
| FGF-18 | (6.3+2.9) × 10⁵ | (0.4±0.30) × 10⁻² | 0.833 | 10.8±9.7 |
| HB-EGF | (6. 6±0.4) × 10⁵ | (0.3±0.10) × 10⁻² | 0.996 | 4.7±3.7 |
| MK | (3.5±1.5) × 10⁵ | (0.6±0.08) × 10⁻² | 0.853 | 204.0±100.0 |
| PTN | (2.6±0.9) × 10⁵ | (0.3±0.20) × 10⁻² | 0.902 | 16.1±13.3 |

| CH-S | | | | |
|---|---|---|---|---|
| FGF-2 | (7.7±1.4) × 10⁵ | (2.3±0.6) × 10⁻² | - | 30.5±7.8 |
| FGF-10 | (9.7±5.1) × 10³ | (4.5±3.5) × 10⁻⁶ | - | 0.4±0.2 |
| FGF-18 | (3.3 ±0.6) × 10⁵ | (7.0±0.9) × 10⁻⁵ | - | 0.2±0.08 |
| HB-EGF | (1.1-±-0.6) × 10⁶ | (9.1±0.4) × 10⁻³ | - | 10.0±4.0 |
| MK | (8.2±3.4) × 10⁴ | (8.9±2,0) × 10⁻⁵ | - | 1.5±0.9 |
| PTN | (4.2±0.3) × 10⁵ | (7.4±2.5) × 10⁻⁵ | - | 0.17±0.05 |

This difference may be due to the structural difference of the SS-CS/DS preparation used, suggesting the importance of the hybrid structure of SS-CS/DS for PTN binding. This idea is consistent with the recent findings on CS/DS chains derived from embryonic pig brain (Bao, X et al., (2004) J. Biol. Chem. 79, 9765-9776).

Interestingly, the affinity exhibited by SS-CS/DS (1.5 M) to the various growth factors was higher than that by Hep to the growth factors except for FGF-2 and HB-EGF (Table 4). The high binding capacity, affinity, and specificity of SS-CS/DS to these growth factors suggest the importance of the CS/DS hybrid structure.

### <Procedures>

### Example 5 Interaction of SS-CS/DS (1.5 M) with BDNF and GDNF

The interaction analysis of SS-CS/DS (1.5 M) with BDNF and GDNF was conducted with BIAcore J system (BIAcore AB, Uppsala, Sweden). Biotinylated SS-CS/DS (1.5 M) was immobilized on sensor chips coated with streptavidin, onto which BDNF and GDNF were then separately injected at varying concentrations (high flow rate of 60 µl/min), for 2 minutes in an association phase and for 3 minutes in a dissociation phase. Kinetic parameters kₐ, k_{d}, and K_{d} were calculated with BIA evaluation 3.1 software.

### <Result>

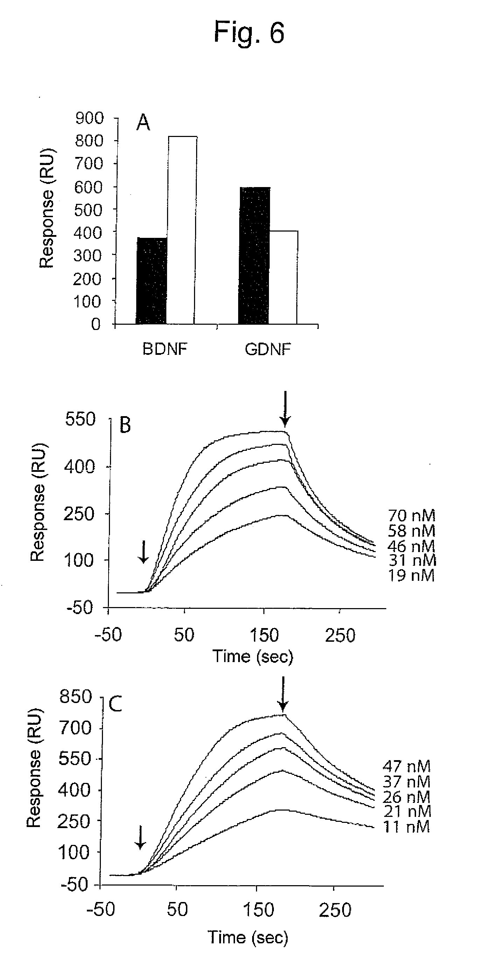

As a result of test with an excessive amount of BDNF and GDNF, S-CS/DS (1.5 M) more highly bonded to GDNF than BDNF (Figure 6A).

Even though the binding reaction of SS-CS/DS (1.5 M) to GDNF was approximately 1.5- to 2.2-fold higher than that of CS-H (control), the binding capacity of SS-CS/DS (1.5 M) to BDNF or GDNF was remarkably high (7.9- and 19.0-fold, respectively) as compared with that of CS-H. The result is shown in Table 5.

**Table 5**

| Growth factor | Average molecular weight | Molar ratio of binding | | | Binding capacity | | |
|---|---|---|---|---|---|---|---|
| | | SS-CS/DS(1.5M) | CS-H | Hep | SS-CS/DS(1.5 M) | CS-H | Hep |
| | (kDa) | (mole/mole) | | | (mole/30 disaccharides) | | |
| BDNF | 13.6 | 11.1 | 1.4 | ND | 2.4 | 1.3 | ND |
| GDNF | 20.0 | 11.4 | 0.6 | ND | 2.5 | 0.5 | ND |

### Example 6 Study on neurite growth-promoting activity of three purified SS-CS/DS preparations (Native, 1.0 M, and 1.5 M)

### <Procedures>

This study was conducted as previously described (Hikino, M et al., (2003) J. Biol. Chem. 278, 43744-43754; and Fassiner, A et al., (1994) J. Cell Biol. 126, 783-799). Coverslips were coated with 600 µl of 1.5 µg/ml poly-DL-ornithine (P-ORN) at 37°C for 2 hours, to which three SS-CS/DS preparations (Native, 1.0 M, and 1.5 M) at 2 µg/well or equal amounts of chondroitinase ABC, AC-I, or B digests of the SS-CS/DS preparations were then applied and incubated overnight at 37°C. Chondroitin sulfate E derived from squid cartilage was used as a positive control.

Hippocampal neurons isolated from E16 mouse embryos were dispersed at a density of 10,000 cells/mm² over the coverslips and cultured in an Eagle's minimum essential medium at 37°C for 24 hours under 5% CO₂. Then, the cells were fixed and subjected to immunostaining using monoclonal antibodies against neurofilament (NF) and microtubule-associated protein (MAP2).

The immunostained cells on each coverslip were counted under a microscope. The length of the longest neurite and the number of neurites of randomly selected cells were determined with image analysis software (Mac SCOPE; Mitani Corp., Fukui, Japan).

### <Result>

When neurite outgrowth-promoting activity is indicated by the length of the longest elongated neurite, neurite outgrowth promotion was observed in the three SS-CS/DS preparations (Native, 1.0 M, and 1.5 M), all of which exceeded CS-E (Figure 7-1A).

Neurite outgrowth-promoting activity was highest in SS-CS/DS (Native), followed by SS-CS/DS (1.5 M), and SS-CS/DS (1.0 M).

The neurons cultured on the coverslip coated with SS-CS/DS (Native) were observed to elongate neurites (Figure 7-2D) as compared with the cells (Figure 7-2E) cultured on the coverslip coated with P-ORN alone as a control.

The cells cultured on the three SS-CS/DS preparations (Native, 1.0 M, and 1.5 M) had 3 or more neurites per cell on average (Figure 7-1C).

To examine the relationship between SS-CS/DS (Native) structure and neurite outgrowth-promoting activity, the chondroitinase ABC, AC-I, or B digests were evaluated for neurite outgrowth-promoting activity. As a result, all the digests lacked neurite outgrowth-promoting activity (Figure 7-1B). Thus, both CS-like and DS-like structures are considered to participate in the activity.

### Example 7 Anti-factor IIa activity measurement

### <Procedures>

Anti-factor IIa activity was measured by incubating 1 to 10 µg/ml of three SS-CS/DS preparations (Native, 1.0 M, and 1.5 M) in 100 µl of 50 mM Tris-HCl buffer (pH 8.3) containing 227 mM NaCl, with 60 µl of normal human plasma and 40 µl of human thrombin (1.2 NIH units/ml) at 25°C for 1 minute (Sakai, S et al., (2003) Carbohydr. Res. 338, 263-269). The amidolytic activity of thrombin was determined by measuring a change in absorbance at 405 nm for 100 seconds after the addition of 100 µl of 1.9 µmol/ml chromozym TH. The rate of change in absorbance was proportional to the residual thrombin activity. Pig skin-derived heparin and dermatan sulfate were used as positive controls.

### <Result>

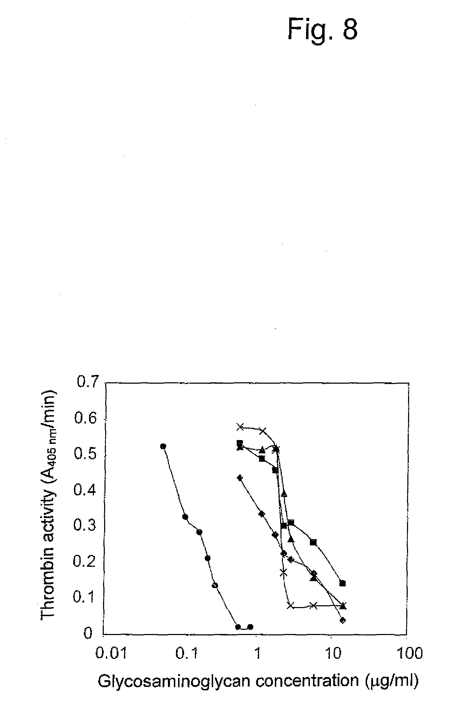

The three SS-CS/DS preparations (Native, 1.0 M, and 1.5 M) exhibited thrombin activity inhibition equivalent to that of the positive controls. SS-CS/DS (Native) exhibited slightly higher anti-factor IIa activity than that of other two SS-CS/DS preparations (1.0 M and 1.5 M) (Figure 8).

### Example 8 Separation of chondroitin sulfate/dermatan sulfate hybrid chain fraction from hagfish notochord

### <Procedures>

The notochord of hagfish *Eptatretus burgeri* was treated with acetone and digested by adding pronase to the suspension. Proteins were precipitated by means of the addition of 20% trichloroacetic acid and subsequent centrifugation, and the supernatant was collected. Glycosaminoglycan was precipitated by adding 2 volumes of ethanol containing 2.5% calcium acetate. The glycosaminoglycan mixture was passed through a Dowex 1-Cl⁻ column and eluted stepwise with a NaCl solution. Heparan sulfate was removed from the 2.0 M NaCl eluted fraction according to the method of Shively and Conrad. After nitrous acid treatment, the nitrous acid was neutralized with 0.5 M Na₂CO₃, and the heparan sulfate fragment was desalted with a Sephadex G-50 column (1×56 cm). To remove free peptides from the desalted glycosaminoglycan fraction, it was passed through a Sep-Pak C 18 cartridge column and eluted with water.

### <Result>

As a result of purification, the 2.0 M NaCl eluted fraction contained a chondroitin sulfate/dermatan sulfate hybrid chain (CS/DS chain). In this context, the CS/DS chain thus isolated from hagfish *Eptatretus burgeri* notochord is referred to as HN-CS/DS.

### Example 9 Molecular weight determination

### <Procedures>

The molecular size of the chondroitin sulfate/dermatan sulfate (CS/DS) hybrid chain was measured by gel filtration using a Superdex 200 column (10×300 mm). HN-CS/DS was loaded onto the column and eluted with 0.2 M ammonium acetate at a flow rate of 0.3 ml/min. The fractions were collected at 3-minute intervals, then evaporated to dryness, and dissolved with 100 µl of water. The measurement was performed according to the previous report (Chandrasekhar, S., et al (1987) Anal Biochem. 161, 103-108).

### <Result>

When the HN-CS/DS molecule was examined by gel filtration column chromatography, it exhibited an average molecular weight of 18 kDa, which was similar to those of pig skin-derived dermatan sulfate (19 kDa) and eel skin-derived dermatan sulfate (14 kDa).

### Example 10 Disaccharide composition analysis

### <Procedures>

### Enzymatic digestion

Disaccharide composition analysis was conducted by analyzing a chondroitinase ABC, B, or AC-I digest.

HN-CS/DS (0.5 µg) was digested with either of 10 mIU of chondroitinase ABC, 5 mIU of chondroitinase AC-I, or 1 mIU chondroitinase B, and the respective digests were labeled with 2-aminobenzamide (2AB) according to the previous report (Kinoshita, A., Sugahara, K (1999) Anal. Biochem. 269, 367-378) (excessive 2AB was removed with a water:chloroform (1:1, v/v) mixture).

The disaccharides labeled with 2AB were diluted with 400 µl of 16 mM NaH₂PO₄ and analyzed by anion-exchange HPLC (PA-03 silica column using 16 mM and 530 mM NaH₂PO₄ solvents).

### Gel filtration analysis of chondroitinase digests of HN-CS/DS on Superdex peptide column

HN-CS/DS was digested with chondroitinase ABC, AC-I, or B.

The respective digests were labeled with 2AB and, then dissolved in 0.2 M ammonium bicarbonate containing 7% 1-propanol, and analyzed on a Superdex Peptide column by fluorescence detection (flow rate: 0.4 ml/min).

### <Result>

The main components of HN-CS/DS were ΔHexUAα1-3GalNAc(4S,6S) (ΔDi-diS_{E}) and ΔHexUAα1-3GalNAc(4S) (ΔDi-4S). It also contained ΔHexUAα1-3GalNAc(6S) (ΔDi-6S), ΔHexUAα1-3GalNAc (ΔDi-0S), and ΔHexUA(2S) α1-3GalNAc(4S,6S) (ΔDi-triS) (Table 6).

**Table 6**

| | ABC | B | AC-I |
|---|---|---|---|
| | pmoles (mole%) | | |
| ΔDi-0S | 68.3(4.7) | 4.0(8.7) | - |
| ΔDi-6S | 197.6(13.5) | 13.8(30.0) | 2.7(7.8) |
| ΔDi-4S | 558.0(38.3) | 10.3(22.4) | 25.9(74.6) |
| ΔDi-diS_{E} | 577.5(39.6) | 17.9(38.9) | 6.1(17.6) |
| ΔDi-triS | 56.4(3.9) | ND | ND |
| Total | 1458 | 46.0 | 34.7 |

### Example 11 Interaction analysis with a variety of growth factors

### <Procedures>

The interaction analysis of HN-CS/DS with a variety of growth factors (FGF-2, FGF-10, FGF-16, FGF-18, PTN, MK, HB-EGF, and VEGF) was conducted with BIAcore system (BIAcore, Uppsala, Sweden). Kinetic parameters kₐ, k_{d}, and K_{d} were calculated with BIA evaluation 3.1 software.

### <Result>

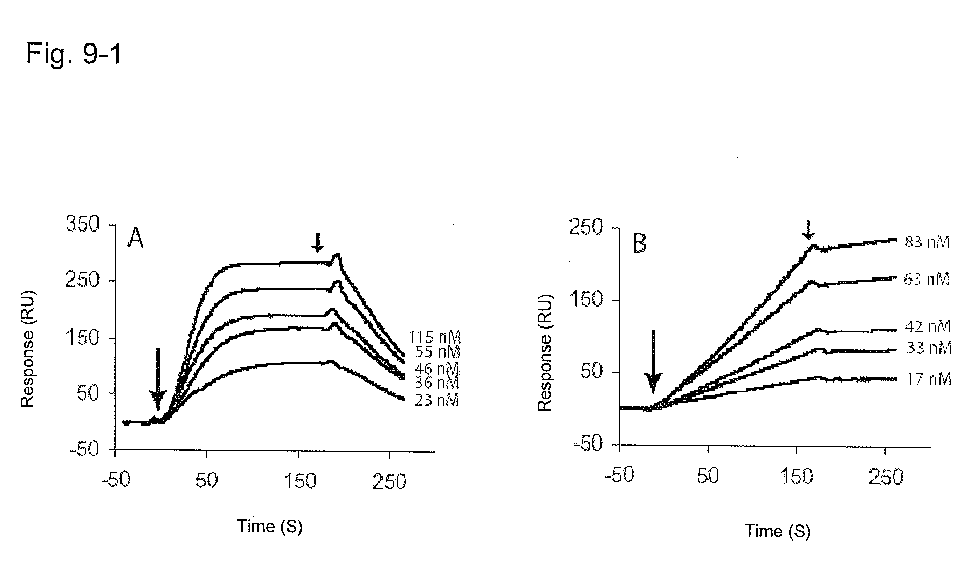

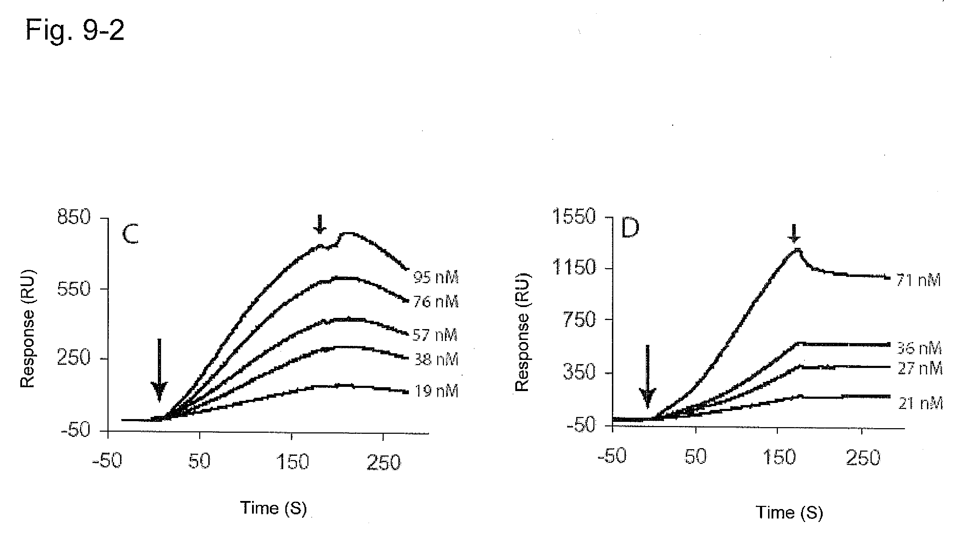

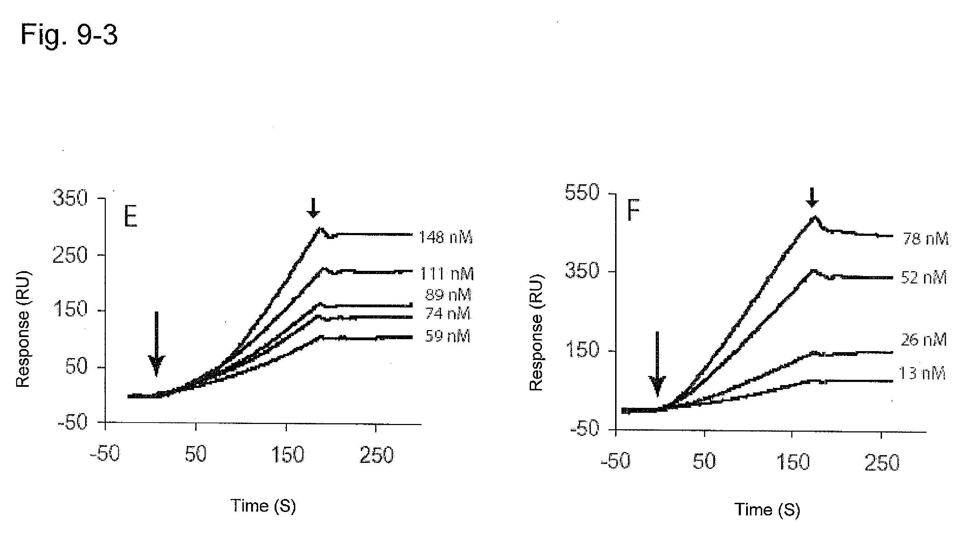

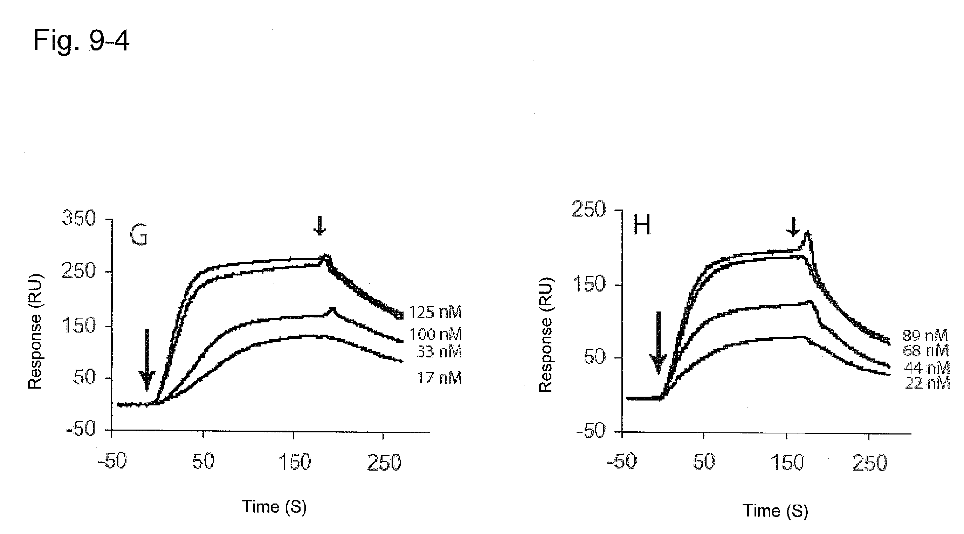

To determine binding affinity, varying concentrations of growth factors were allowed to interact with immobilized HN-CS/DS. Sensorgrams of HN-CS/DS binding to FGF-2 (A), FGF-10 (B) (Figure 9-1), FGF-16 (C), FGF-18 (D) (Figure 9-2), MK (E), PTN (F) (Figure 9-3), HB-EGF (G), and VEGF (H) (Figure 9-4) are shown in Figures 9-1 to 9-4.

All the growth factors tested exhibited binding patterns typical of growth factors.

It was revealed that all the growth factors tested have high affinity to HN-CS/DS (Table 7). Especially FGF-18 (K_{d}=0.2 nM), FGF-10 (K_{d}=0.4 nM), and PTN (K_{d}=0.17 nM) exhibited high affinity.

**Table 7**

| Growth factor | kₐ (M⁻¹ S⁻¹) | k_{d} (S⁻¹) | K_{d} (nM) |
|---|---|---|---|
| FGF-2 | (7.7±1.4) × 10⁵ | (2.3±0.6) × 10⁻² | 30.5±7.8 |
| FGF-10 | (9.7±5.1) × 10³ | (4.5±3.5) × 10⁻⁶ | 0.4±0.2 |
| FGF-16 | (5.6±2.1) × 10⁶ | (6.9±3.1) × 10⁻³ | 1.2±0.8 |
| FGF-18 | (3.3 ±0.6) × 10⁵ | (7.0±0.9) × 10⁻⁵ | 0.2±0.08 |
| HB-EGF | (1.1±0.6) × 10⁶ | (9.1±0.4) × 10⁻³ | 10.0±4.0 |
| MK | (8.2±3.4) × 10⁴ | (8.9±2.0) × 10⁻⁵ | 1.5±0.9 |
| PTN | (4.2±0.3) × 10⁵ | (7.4±2.5) × 10⁻⁵ | 0.17±0.05 |
| VEGF | (7.0±1.6) × 10⁵ | (1.7±0.5) × 10⁻² | 24±7 |

### Example 12 Interaction of HN-CS/DS with neurotrophic factors

### <Procedures>

The interaction analysis of HN-CS/DS with BDNF and GDNF was conducted with BIAcore J system (BIAcore, Uppsala, Sweden). Biotinylated HN-CS/DS was immobilized on sensor chips coated with streptavidin, onto which BDNF and GDNF were then separately injected at varying concentrations (high flow rate of 60 µl/min), for 2 minutes in an association phase and for 3 minutes in a dissociation phase. Kinetic parameters kₐ, k_{d}, and K_{d} were calculated with BIA evaluation 3.1 software (Table 8).

### <Result>

**Table 8**

| | ka(M⁻¹s⁻¹) | kd(s⁻¹) | Kd(nM) |
|---|---|---|---|
| HN-CS/DS | | | |
| BDNF | 4.4×10⁷ | 3.0×10⁻² | 0.7 |
| GDNF | 2.0×10⁶ | 5.2×10⁻³ | 2.5 |

| HS | | | |
|---|---|---|---|
| BDNF | 2.6×10⁵ | 6.7×10⁻² | 254 |
| GDNF | 2.0×10⁶ | 5.2×10⁻³ | 24 |

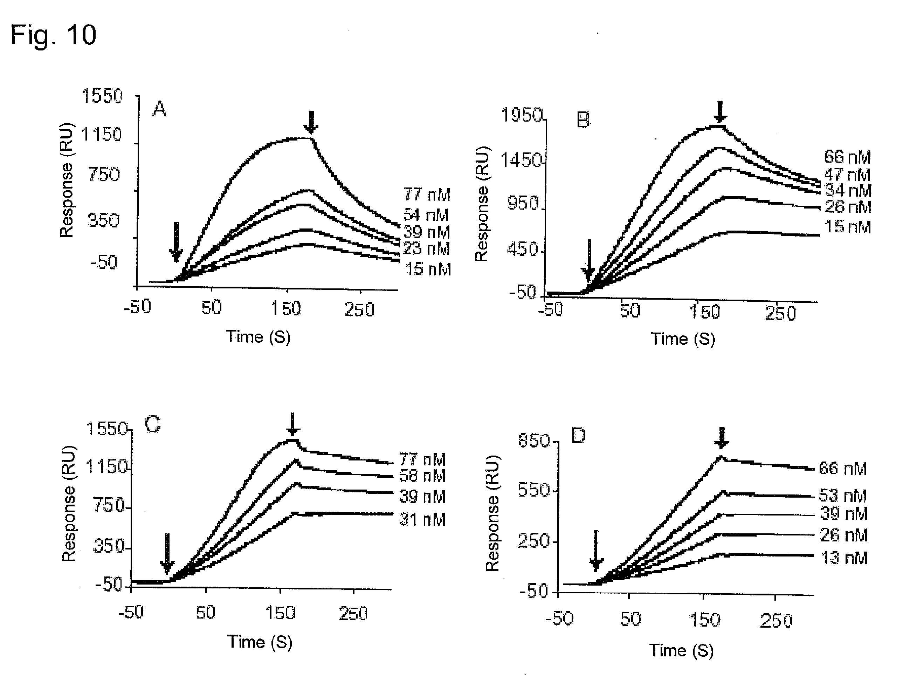

Sensorgrams of varying concentrations of BDNF and GDNF perfused onto the HN-CS/DS-immobilized sensor chips are shown in Figure 10.

The affinity of HN-CSIDS to GDNF was approximately 10-fold higher than that of heparan sulfate (HS) to GDNF, whereas the affinity of HN-CS/DS to BDNF was remarkably higher (360-fold) than that of HS to BDNF. This high binding affinity suggests the importance of chondroitin sulfate, dermatan sulfate, or a CS/DS hybrid structure for binding.

This high binding affinity to the neurotrophic factors was reported for the fist time. Since HN-CS/DS exhibited efficient binding as compared with HS, this hybrid chain is deduced to work in binding manner different from HS and regulate the activity of the neurotrophic factors.

### Example 13 Study on neurite growth-promoting activity

### <Procedures>

This study was conducted as previously described (Hikino, M et al., (2003) J. Biol. Chem. 278, 43744-43754; and Fassiner, A et al., (1994) J. Cell Biol. 126, 783-799). Coverslips were coated with 600 µl of 1.5 µg/ml poly-DL-ornithine (P-ORN) at 37°C for 2 hours, to which HN-CS/DS at 0.7 µg/well or equal amounts of chondroitinase ABC, AC-I, or B digests of HN-CS/DS were then applied and incubated overnight at 37°C. CS-E derived from squid cartilage was used as a positive control.

Hippocampal neurons isolated from E16 mouse embryos were dispersed at a density of 10,000 cells/mm² over the coverslips and cultured in an Eagle's minimum essential medium at 37°C for 24 hours under 5% CO₂. Then, the cells were fixed and subjected to immunostaining using monoclonal antibodies against neurofilament (NF) and microtubule-associated protein (MAP2).

The immunostained cells on each coverslip were counted under a microscope. The length of the longest neurite and the number of neurites of randomly selected cells were determined with image analysis software (Mac SCOPE; Mitani Corp., Tokyo, Japan).

### <Result>

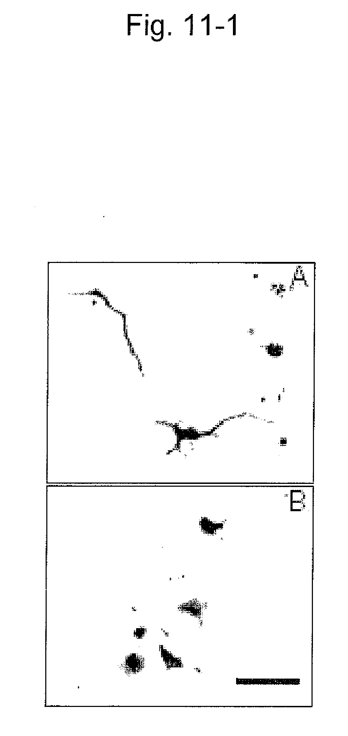

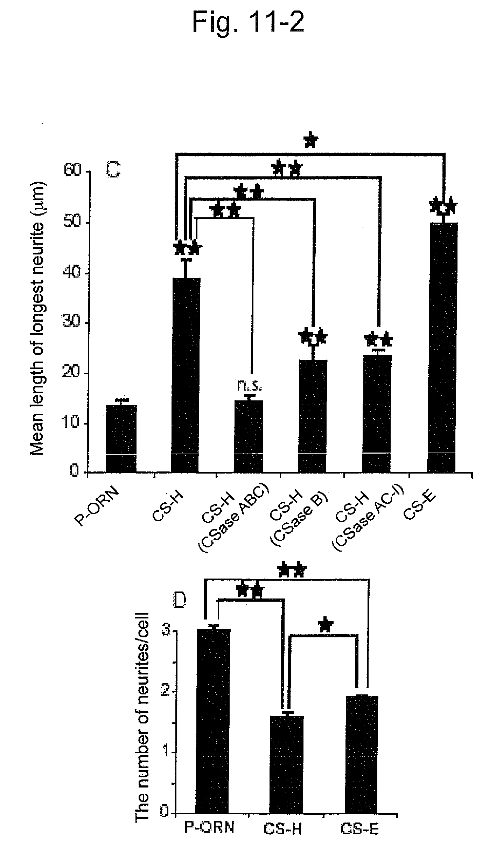

When neurite outgrowth-promoting activity is indicated by the length of the longest elongated neurite, neurite outgrowth promotion was observed in HN-CS/DS as compared with the control (Figure 11-2C).

The neurons cultured on the coverslip coated with HN-CS/DS were observed to elongate neurites (Figure 11-1A) as compared with the cells (Figure 11-1B) cultured on the coverslip coated with P-ORN alone as a control.

To examine the relationship between HN-CS/DS structure and neurite outgrowth-promoting activity, the chondroitinase ABC, AC-I, or B digests were evaluated for neurite outgrowth-promoting activity. As a result, the chondroitinase ABC digest lacked neurite outgrowth-promoting activity. On the other hand, obvious reduction in activity was also observed by chondroitinase AC-I or B digestion (Figure 11-2C), though the degree of reduction in activity was lower than that by chondroitinase ABC digestion. This shows that the CS/DS hybrid structure consisting of glucuronic acid-containing CS-like and iduronic acid-containing DS-like structures participates in the neurite outgrowth-promoting activity.

### Industrial Applicability

According to the present invention, a novel chondroitin sulfate/dermatan sulfate hybrid chain binding to a variety of growth factors and exhibiting neurite outgrowth-promoting activity and anticoagulant activity, and a fraction containing the chain are provided. Compositions for neurological disease treatment containing them are further provided. The chondroitin sulfate/dermatan sulfate hybrid chain of the present invention is derived from fishes except for bony fishes such as shark skin and hagfish, and the useful application of the fishes was found by the present invention.

All publications cited herein are incorporated herein in their entirety. Those skilled in the art will easily understand that various variations and modifications of the present invention may be made therein without departing from technical principles described in the appended claims and the scopes of the present invention. The present invention is intended to encompass such variations and modifications.

## Claims

1. A chondroitin sulfate/dermatan sulfate hybrid chain comprising a disaccharide GlcUA(2S)-GalNAc(4S) (B unit).

2. The chondroitin sulfate/dermatan sulfate hybrid chain according to claim 1, wherein the chain comprises GlcUA(2)-GalNAc(45) (B unit) and further comprises at least one unit selected from the group consisting of GlcUA-GalNAc(4S) (A unit), GlcUA-GalNAc(6S) (C unit), GlcUA(2S)-GalNAc(6S) (D unit), GlcUA-GalNAc(4S,6S) (E unit), and unsulfated GlcUA-GalNAc (O unit) and at least one unit selected from the group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), and IdoUA-GaINAc(4S,6S) (iE unit).

3. The chondroitin sulfate/dermatan sulfate hybrid chain according to claim 1 or 2, wherein an average molecular weight thereof is 65 to 75 kDa.

4. The chondroitin sulfate/dermatan sulfate hybrid chain according to any one of claims 1 to 3, wherein the degree of sulfation per disaccharide molecule falls within the range of 0.70 or more to less than 1.2.

5. The chondroitin sulfate/dermatan sulfate hybrid chain according to any one of claims 1 to 4, wherein the chain is derived from shark skin.

6. Glycosaminoglycan comprising a chondroitin sulfate/dermatan sulfate hybrid chain according to any one of claims 1 to 5.

7. A growth factor-binding agent comprising glycosaminoglycan according to claim 6 as an active ingredient.

8. A neurite outgrowth-promoting agent comprising glycosaminoglycan according to claim 6 as an active ingredient.

9. An anticoagulant comprising glycosaminoglycan according to claim 6 as an active ingredient.

10. A pharmaceutical composition comprising glycosaminoglycan according to claim 6 as an active ingredient.

11. A pharmaceutical composition for neurodegenerative disease prevention or treatment comprising glycosaminoglycan according to claim 6 as an active ingredient.

12. Use of glycosaminoglycan according to claim 6 for producing a growth factor-binding agent, neurite outgrowth-promoting agent, anticoagulant, or composition for neurodegenerative disease treatment.

13. Glycosaminoglycan comprising a chondroitin sulfate/dermatan sulfate hybrid chain derived from fishes except for bony fishes.

14. The glycosaminoglycan comprising a chondroitin sulfate/dermatan sulfate hybrid chain according to claim 13, wherein the chain comprises at least one unit selected from the group consisting of GlcUA-GalNAc(4S) (A unit), GlcUA(2S)-GalNAc(4S) (B unit), GlcUA-GalNAc(6S) (C unit), GlcUA(2S)-GalNAc(6S) (D unit), GlcUA-GalNAc(4S,6S) (E unit), GlcUA(2S) β1-3GalNAc(4S,6S) (T unit), and unsulfated GlcUA-GalNAc (O unit) and at least one unit selected from the group consisting of IdoUAα1-3GalNAc(4S) (iA unit), IdoUA(2S)-GalNAc(4S) (iB unit), IdoUAα1-3GalNAc(6S) (iC unit), IdoUA(2S)-GalNAc(6S) (iD unit), IdoUA-GalNAc(4S,6S) (iE unit), and IdoUA(2S) α1-3GalNAc(4S,6S) (iT unit).

15. A growth factor-binding agent comprising glycosaminoglycan according to claim 13 or 14 as an active ingredient.

16. A neurite outgrowth-promoting agent comprising glycosaminoglycan according to claim 13 or 14 as an active ingredient.

17. A composition for neurodegenerative disease treatment comprising glycosaminoglycan according to claim 13 or 14 as an active ingredient.

18. A pharmaceutical composition comprising glycosaminoglycan according to claim 13 or 14 as an active ingredient.

19. A pharmaceutical composition for the prevention or treatment of neurodegenerative disease comprising glycosaminoglycan according to claim 13 or 14 as an active ingredient.

20. Use of glycosaminoglycan according to claim 13 or 14 for producing a growth factor-binding agent, neurite outgrowth-promoting agent, or composition for neurodegenerative disease treatment.
